# EUROPEAN PATENT SPECIFICATION

(11) **EP 2 637 687 B1**
(45) Date of publication and mention of the grant of the patent: **06.01.2021**
(21) Application number: 11785541.1
(22) Date of filing: 07.11.2011
(51) Int. Cl.: A61K 39/008, C07K 14/44, C12N 15/62

(54) **VACCINES COMPRISING NON-SPECIFIC NUCLEOSIDE HYDROLASE AND STEROL 24-C-METHYLTRANSFERASE (SMT) POLYPEPTIDES FOR THE TREATMENT AND DIAGNOSIS OF LEISHMANIASIS**
IMPFSTOFFE MIT NICHTSPEZIFISCHER NUKLEOSIDHYDROLASE UND STEROL-24-C-METHYLTRANSFERASE (SMT)-POLYPEPTIDEN ZUR BEHANDLUNG UND DIAGNOSE VON LEISHMANIOSE
VACCINS COMPRENANT DES POLYPEPTIDES D'HYDROLASE NUCLÉOSIDIQUE ET DE STÉROL 24-C-MÉTHYLTRANSFÉRASE (SMT) NON SPÉCIFIQUES DESTINÉS À TRAITER ET À DIAGNOSTIQUER LA LEISHMANIOSE

(30) Priority: 08.11.2010 US 411366 P
(43) Date of publication of application: 18.09.2013
(73) Proprietor: Infectious Disease Research Institute, Seattle, Washington 98102 (US)
(72) Inventor: BHATIA, Ajay, Seattle, Washington 98118 (US); REED, Steven, G., Bellevue, Washington 98005 (US)
(74) Representative: Mewburn Ellis LLP
(86) International application number: PCT/US2011/059600
(87) International publication number: WO 2012/064659

(56) References cited:
- WO-A1-2009/143006
- Reed S: "Next Generation Vaccines Against Leishmaniasis", , 11 October 2010 (2010-10-11), XP002670762, Retrieved from the Internet: URL:http://ec.europa.eu/research/health/in fectious-diseases/neglected-diseases/pdf/n id-conference/steve-reed-ec-leish3_en.pdf [retrieved on 2012-03-01]
- ZANIN ET AL: "Evaluation of immune responses and protection induced by A2 and nucleoside hydrolase (NH) DNA vaccines against Leishmania chagasi and Leishmania amazonensis experimental infections", MICROBES AND INFECTION, ELSEVIER, PARIS, FR, vol. 9, no. 9, 23 August 2007 (2007-08-23) , pages 1070-1077, XP022211642, ISSN: 1286-4579, DOI: 10.1016/J.MICINF.2007.05.012
- SANTANA DEBORA M ET AL: "Nucleoside hydrolase from Leishmania (L.) donovani is an antigen diagnostic for visceral leishmaniasis", MOLECULAR AND BIOCHEMICAL PARASITOLOGY, vol. 120, no. 2, 9 April 2002 (2002-04-09) , pages 315-319, XP002670855, ISSN: 0166-6851
- PARAGUAI DE SOUZA E ET AL: "Vaccination of Balb/c mice against experimental visceral leishmaniasis with the GP36 glycoprotein antigen of Leishmania donovani", VACCINE, ELSEVIER LTD, GB, vol. 19, no. 23-24, 30 April 2001 (2001-04-30), pages 3104-3115, XP004234181, ISSN: 0264-410X, DOI: 10.1016/S0264-410X(01)00031-7
- GOTO ET AL: "Protective immunization against visceral leishmaniasis using Leishmania sterol 24-c-methyltransferase formulated in adjuvant", VACCINE, ELSEVIER LTD, GB, vol. 25, no. 42, 28 September 2007 (2007-09-28), pages 7450-7458, XP022277624, ISSN: 0264-410X, DOI: 10.1016/J.VACCINE.2007.08.001
- SKEIKY Y A W ET AL: "Protective efficacy of a tandemly linked, multi-subunit recombinant leishmanial vaccine (Leish-111f) formulated in MPL(R) adjuvant", VACCINE, ELSEVIER LTD, GB, vol. 20, no. 27-28, 10 September 2002 (2002-09-10), pages 3292-3303, XP004378524, ISSN: 0264-410X, DOI: 10.1016/S0264-410X(02)00302-X
- KUMAR RAJIV ET AL: "Evaluation of Ex Vivo Human Immune Response against Candidate Antigens for a Visceral Leishmaniasis Vaccine", AMERICAN JOURNAL OF TROPICAL MEDICINE AND HYGIENE, vol. 82, no. 5, May 2010 (2010-05), pages 808-813, XP002670763,
- DUTHIE MALCOLM S ET AL: "The development and clinical evaluation of second-generation leishmaniasis vaccines.", VACCINE 5 JAN 2012 LNKD- PUBMED:22085553, vol. 30, no. 2, 5 January 2012 (2012-01-05), pages 134-141, XP002670764, ISSN: 1873-2518
- COREEN M. BEAUMIER ET AL: "New vaccines for neglected parasitic diseases and dengue", TRANSLATIONAL RESEARCH, vol. 162, no. 3, 1 September 2013 (2013-09-01), pages 144-155, XP55186067, ISSN: 1931-5244, DOI: 10.1016/j.trsl.2013.03.006

## Description

### BACKGROUND

### Technical Field

The present invention relates generally to compositions and methods for preventing, treating and detecting leishmaniasis in patients. More particularly, the invention relates to compositions and methods comprising *Leishmania* antigens and fusion polypeptides, as well as polynucleotides encoding such antigens and fusion polypeptides.

### Description of the Related Art

*Leishmania* organisms are obligate intracellular parasites that cause a large clinical spectrum of diseases named leishmaniasis. *Leishmania* organisms are intracellular protozoan parasites of the genus *Leishmania. Leishmania* organisms target host macrophages; thus causing a wide spectrum of clinical diseases in humans and domestic animals, primarily dogs. In some infections, the parasite may lie dormant for many years. In other cases, the host may develop one of a variety of forms of leishmaniasis. Leishmaniases are roughly classified into three types of diseases, cutaneous leishmaniasis (CL), mucosal leishmaniasis (ML) and visceral leishmaniasis (VL), according to the clinical manifestations.

Leishmaniasis is a serious problem in much of the world, including Brazil, China, East Africa, India and areas of the Middle East. The disease is also endemic in the Mediterranean region, including southern France, Italy, Greece, Spain, Portugal and North Africa. The number of cases of leishmaniasis has increased dramatically in the last 20 years, and millions of cases of this disease now exist worldwide. About 2 million new cases are diagnosed each year, 25% of which are visceral leishmaniasis.

Visceral leishmaniasis (VL) has been reported in 88 countries, but roughly 90% of VL cases occur in Brazil, India, Sudan, Bangladesh, and Nepal (Mendez et al. J Immunol 2001; 166(8): pp. 5122-8). The annual incidence is estimated to be approximately 500,000 cases of VL, and the population at risk is 350 million (Engwerda et al. Eur J Immunol 1998; 28(2): pp. 669-80; Squires et al. J Immunol 1989; 143(12): pp. 4244-9). Visceral leishmaniasis, generally caused by species of the *L. donovani* complex, i.e. *L. donovani* and *L. infantum* (*chagasi*). *L. donovani* is the causative agent of visceral leishmaniasis in Africa and Asia, *L. infantum*/*chagasi* in Mediterranean countries and in the New World (Piedrafita et al. J Immunol 1999; 163(3): pp. 1467-72). VL is a severe debilitating disease that evolves with visceral infection involving the spleen, liver and lymph nodes, which, untreated, is generally a fatal disease. Symptoms of acute visceral leishmaniasis include hepatosplenomegaly, fever, leukopenia, anemia and hypergammaglobulinemia. Active VL is generally fatal unless properly treated.

*Leishmania* parasites are transmitted by the bite of sandflies and the infecting promastigotes differentiate into and replicate as amastigotes within macrophages in the mammalian host. In common with other intracellular pathogens, cellular immune responses are critical for protection against leishmaniasis. Th1 immune responses play an important role in mediating protection against *Leishmania,* including roles for CD4⁺ and CD8⁺ T cells, IFN-γ, IL-12, TNF-α and NO, whereas inhibitory effects have been reported for IL-10 and TGF-β (Engwerda et al. Eur J Immunol 1998; 28(2): pp. 669-80; Murphy et al. Eur J Immunol. 2001; 31(10): pp. 2848-56; Murray et al. J Exp Med. 1999; 189(4): pp.741-6; Murray et al. Infect Immun. 2000; 68(11): pp. 6289-93; Squires et al. J Immunol 1989; 143(12): pp. 4244-9 6; Taylor and Murray. J Exp Med. 1997; 185(7): pp. 1231-9; Kaye and Bancroft. Infect Immun. 1992; 60(10): pp. 4335-42; Stern et al. J Immunol. 1988; 140(11): pp. 3971-7; Wilson et al. J Immunol. 1998; 161(11): pp. 6148-55).

Immunization against leishmaniasis in animal models can be effected by delivery of antigen-encoding DNA vectors (Gurunathan et al. J Exp Med. 1997; 186(7): pp. 1137-47; Piedrafita et al. J Immunol. 1999;163(3):1467-72; Mendez et al. J Immunol. 2001; 166(8): pp. 5122-8) or by administration of proteins formulated with Th1-inducing adjuvants including IL-12 (Afonso etal. Science. 1994; 263(5144): pp. 235-7; Stobie et al. Proc Natl Acad Sci U S A. 2000; 97(15): pp. 8427-32; Kenney et al. J Immunol. 1999; 163(8): pp. 4481-8) or TLR ligands such as CpG oligonucleotides (Rhee et al. J Exp Med. 2002; 195(12): pp. 1565-73; Stacey and Blackwell. Infect Immun. 1999; 67(8): pp. 3719-26; Walker et al. Proc Natl Acad Sci USA. 1999; 96(12): pp. 6970-5) and monophosphoryl lipid A (Coler et al. Infect Immun. 2002; 70(8): pp. 4215-25; Skeiky et al. Vaccine. 2002; 20(2728): pp. 3292-303).

WO2009/143006 discloses compositions and methods for preventing, treating and detecting leishmaniasis. The compositions, generally comprise fusion polypeptides comprising multiple *Leishmania* antigens, in particular, KMP11, SMT, A2 and/or CBP, or immunogenic portions or variants thereof, as well as polynucleotides encoding such fusion polypeptides.

In spite of some evidence that sub-unit vaccines may be effective in certain models of VL (Basu et al. J Immunol. 2005; 174(11): pp. 7160-71; Stager et al. J Immunol. 2000; 165(12): pp. 7064-71; Ghosh et al. Vaccine. 2001; 20(12): pp. 59-66; Wilson et al. Infect Immun. 1995; 63(5): pp. 2062-9; Tewary et al. J Infect Dis. 2005; 191(12): pp. 2130-7; Aguilar-Be et al. Infect Immun. 2005; 73(2): pp. 812-9. Rafati et al. Vaccine. 2006; 24(12):2169-75), progress toward defining antigen candidates effective against VL *in vivo* has been lacking.

Strategies employing vaccines consisting of whole organisms for preventing or treating leishmaniasis have not been effective in humans. In addition, more effective reagents are needed for accurately diagnosing leishmaniasis in patients. Accordingly, there remains a significant need for immunogenic compositions and vaccines that can effectively prevent, treat and/or diagnose leishmaniasis in humans and other mammals (*e.g.,* canines). The present invention fulfills these needs and offers other related advantages

### BRIEF SUMMARY

The invention is set out in the accompanying claims.

Briefly stated, the present invention provides compositions, kits and methods for preventing, treating and detecting leishmaniasis. Therefore, according to one aspect of the invention, there are provided fusion polypeptides comprising at least a *Leishmania* sterol 24-c-methyltransferase (SMT) polypeptide sequence and a Leishmania non-specific nucleoside hydrolase (NH) polypeptide sequence, as set out in the claims.

For example, in certain embodiments, a fusion polypeptide as described herein comprises sequences having at least an immunogenic portion of an SMT protein and at least an immunogenic portion of an NH protein as set out in the claims.

A *Leishmania* NH sequence used in a fusion polypeptide includes a sequence having at least 90% identity to a *Leishmania* NH sequence of *L*. *donovani, L. infantum and L. major.* A *Leishmania* NH polypeptide sequence used in a fusion polypeptide of the invention comprises at least an immunogenic portion of a sequence selected from the group consisting of SEQ ID NOs: 1, 3 and 5, or a sequence having at least 90% identity thereto as set out in the claims.

A *Leishmania* SMT polypeptide sequence used in a fusion polypeptide or polypeptide combination of the invention comprises at least an immunogenic portion of a sequence having at least 90% identity to a *Leishmania* SMT sequence of *L. donovani, L. infantum and L. major.* A *Leishmania* SMT sequence used in a fusion polypeptide or polypeptide combination of the invention comprises at least an immunogenic portion of a sequence selected from the group consisting of SEQ ID NOs: 7, 9 and 11, or a sequence having at least 90% identity thereto as set out in the claims.

In a more specific embodiment, a fusion polypeptide or polypeptide combination of the invention comprises a sequence selected from the group consisting of SEQ ID NO: 1, 3 and 5, and further comprises a *Leishmania* SMT polypeptide sequence selected from the group consisting of SEQ ID NO: 7, 9 and 11.

In an even more specific embodiment, a fusion polypeptide or polypeptide combination of the invention comprises an amino acid sequence set forth in SEQ ID NO: 13, or a sequence having at least 90% identity thereto.

In another aspect of the invention, there is provided an isolated polynucleotide encoding a fusion polypeptide as set out in the claims.

Furthermore, according to another aspect of the invention, there is provided a composition comprising at least one component selected from a fusion polypeptide and/or a polynucleotide encoding a fusion polypeptide or polypeptide combination as set out in the claims, in combination with at least one immunostimulant. Many immunostimulants are known and can be used in the compositions herein, illustrative examples of which include, but are not limited to, a CpG-containing oligonucleotide, synthetic lipid A, MPL™, 3D-MPL™, saponins, saponin mimetics, AGPs, Toll-like receptor agonists, or a combination thereof. Other illustrative immunostimulants comprise, for example, aTLR4 agonist, a TLR7/8 agonist and/or a TLR9 agonist. Still other immunostimulants comprise, for example, imiquimod, gardiquimod and/or resiquimod.

According to yet another aspect of the invention, there is provided a method for stimulating an immune response against *Leishmania* in a mammal comprising administering to a mammal in need thereof a composition as set out in the claims.

In yet another aspect of the invention, there is provided a method for detecting *Leishmania* infection in a biological sample, comprising: (a) contacting a biological sample with a fusion polypeptide as set out in the claims; and (b) detecting in the biological sample the presence of antibodies that bind to the fusion polypeptide, thereby detecting *Leishmania* infection in a biological sample. Any suitable biological sample type may be analyzed by the method, illustrative examples of which may include, for example, sera, blood and saliva.

In certain embodiments of the disclosed diagnostic methods, the fusion polypeptide is bound to a solid support. Accordingly, the present invention further provides diagnostic reagents comprising a fusion polypeptide as described herein, immobilized on a solid support.

Diagnostic kits for detecting *Leishmania* infection in a biological sample are also provided, generally comprising a fusion polypeptide as described herein and a detection reagent. It will be understood that the kit may employ a fusion polypeptide or polypeptide combination of the invention in any of a variety of assay formats known in the art, including, for example, a lateral flow test strip assay, a dual path platform (DPP) assay and an ELISA assay. These kits and compositions of the invention can offer valuable point of care diagnostic information. Furthermore, the kits and compositions can also be advantageously used as test-of-cure kits for monitoring the status of infection in an infected individual over time and/or in response to treatment.

These and other aspects of the present invention will become apparent upon reference to the following detailed description and attached drawings.

### BRIEF DESCRIPTION OF THE DRAWINGS

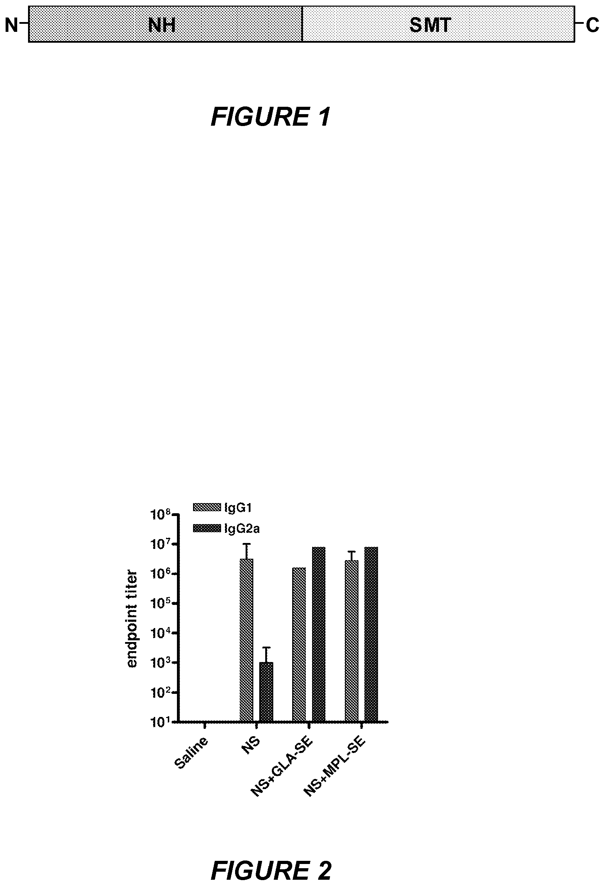

Figure 1 shows a schematic of an illustrative *Leishmania* NS fusion polypeptide.
Figure 2 shows NS specific IgG1 & IgG2a endpoint antibody titers. Mice were injected i.m. three times 3 weeks apart with saline, NS (10 µg) antigen alone, NS (10 µg) + GLA-SE (5 µg) or NS (10 µg) + MPL-SE (20 µg). Serum was collected from immunized mice 3 weeks following the third immunization. Mean reciprocal dilutions are represented as the endpoint titer (Log₁₀) +/- SE.

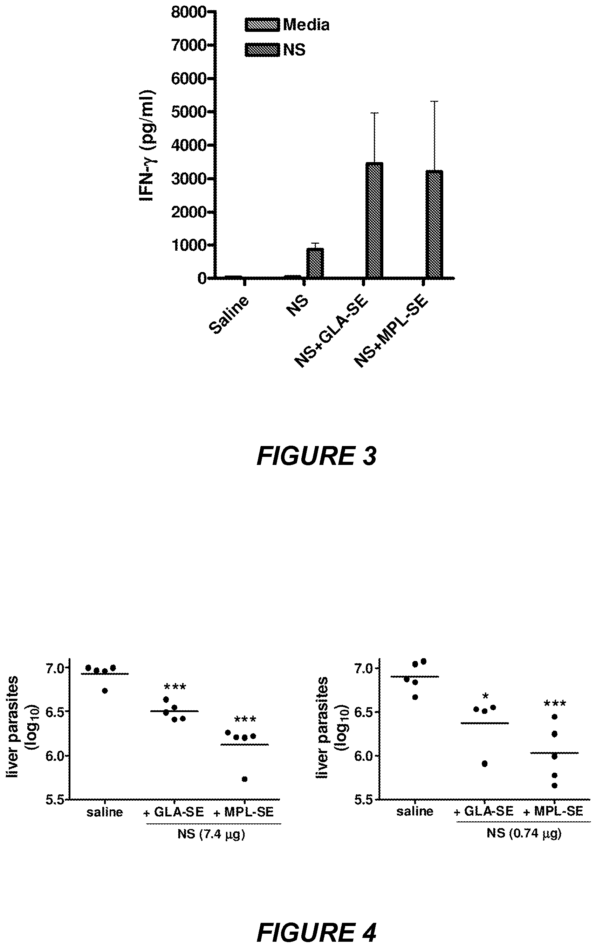

Figure 3 shows NS specific INF-gamma T cell responses in Balb/c mice. Mice were injected i.m. three times 3 weeks apart with saline, NS antigen alone (10 µg), NS (10 µg) + GLA-SE (5 µg) or NS (10 µg) + MPL-SE (20 µg). Splenocytes harvested 3 weeks post last-boost were cultured with medium or fusion protein NS (10 µg/ml) for 72 hours. Culture supernatants were analysed for IFN-γ production by a sandwich ELISA.
Figure 4 shows protection against Leishmania donovani challenge in a Balb/c disease model. BALB/c mice were immunized s.c. 3 times 3 weeks apart with either 7.4 µg of NS (a) or 0.74 µg of NS (b) formulated with either GLA-SE (5 µg) or MPL-SE (20µg). Mice were challenged i.c. with 5x10⁶ *L. donovani* promastigotes one month post last boost and livers harvested one month post-challenge. Parasite burdens were estimated by a limiting dilution assay. The individual and mean parasite numbers (log₁₀) from each group of mice is shown. * *P*<0.05, *** *P*<0.001 by unpaired *t*-test compared to the saline group.

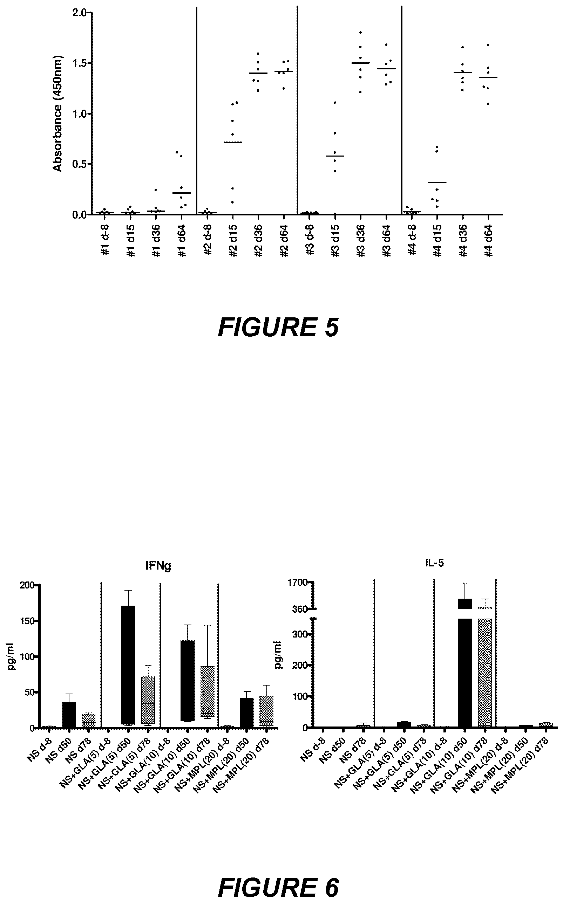

Figure 5 shows NS specific IgG antibody titers determined by ELISA in different groups of monkeys. Antibodies to NS were measured at baseline (d-8) and then again at d15, d36 and d64. The experimental groups have been numbered as listed in Table I.
Figure 6 shows development of vaccine-induced T-cell immune responses. Supernatants from WBA were analyzed for IFN-γ and IL-5 production using the Milliplex assay.

### BRIEF DESCRIPTION OF THE SEQUENCE IDENIFIERS

SEQ ID NO: 1 is an amino acid sequence for a *L. infantum* full-length non-specific nucleoside hydrolase (NH) polypeptide.
SEQ ID NO: 2 is a nucleic acid sequence encoding the polypeptide of SEQ ID NO: 1.
SEQ ID NO: 3 is an amino acid sequence for a *L. donovani* full-length non-specific nucleoside hydrolase (NH) polypeptide.
SEQ ID NO: 4 is a nucleic acid sequence encoding the polypeptide of SEQ ID NO: 3.
SEQ ID NO: 5 is an amino acid sequence for a *L. major* full-length non-specific nucleoside hydrolase (NH) polypeptide.
SEQ ID NO: 6 is a nucleic acid sequence encoding the polypeptide of SEQ ID NO: 5.
SEQ ID NO: 7 is an amino acid sequence for a *L. infantum* full-length sterol 24-c-methyltransferase (SMT) polypeptide.
SEQ ID NO: 8 is a nucleic acid sequence encoding the polypeptide of SEQ ID NO: 7.
SEQ ID NO: 9 is an amino acid sequence for a *L. donovani* full-length sterol 24-c-methyltransferase (SMT) polypeptide.
SEQ ID NO: 10 is a nucleic acid sequence encoding the polypeptide of SEQ ID NO: 9.
SEQ ID NO: 11 is an amino acid sequence for a *L. major* full-length sterol 24-c-methyltransferase (SMT) polypeptide.
SEQ ID NO: 12 is a nucleic acid sequence encoding the polypeptide of SEQ ID NO: 11.
SEQ ID NO: 13 is an amino acid sequence for an illustrative NS fusion polypeptide of the invention.
SEQ ID NO: 14 is a nucleic acid sequence encoding the polypeptide of SEQ ID NO: 13.

### DETAILED DESCRIPTION

The practice of the present invention will employ, unless otherwise indicated, conventional techniques of molecular biology, recombinant DNA, and chemistry, which are within the skill of the art. Such techniques are explained fully in the literature. *See, e.g.,* Molecular Cloning A Laboratory Manual, 2nd Ed., Sambrook et al., ed., Cold Spring Harbor Laboratory Press: (1989); DNA Cloning, Volumes I and II (D. N. Glover ed., 1985); Oligonucleotide Synthesis (M. J. Gait ed., 1984); Mullis et al., U.S. Pat. No: 4,683,195; Nucleic Acid Hybridization (B. D. Hames & S. J. Higgins eds. 1984); B. Perbal, A Practical Guide To Molecular Cloning (1984); the treatise, Methods In Enzymology (Academic Press, Inc., N.Y.); and in Ausubel et al., Current Protocols in Molecular Biology, John Wiley and Sons, Baltimore, Maryland (1989).

The invention is set out in the claims.

As noted above, the present invention is generally directed to compositions and methods for preventing, treating and detecting leishmaniasis. The compositions of the invention include, for example, fusion polypeptides and polypeptide combinations that comprise *Leishmania* sterol 24-c-methyltransferase (SMT) and non-specific nucleoside hydrolase (NH) polypeptides, or immunogenic portions or variants thereof, wherein the portions and variants preferably retain substantially the same or similar immunogenic properties as a corresponding full length SMT and/or NH polypeptide, or a fusion polypeptide or polypeptide combination thereof. Immunization strategies using compositions of the invention can be applied to the *in vivo* protection against, for example, *L. infantum, L. donovani,* and *L. major,* which are causative agents of VL in humans and dogs. The present invention also contemplates, in other embodiments, using the fusion polypeptides and polypeptide combinations described herein in diagnostic applications, including, but not limited to, serodiagnosis and whole blood assays in patients and dogs, preferably in a format amenable to providing rapid, point of care diagnostic results, such as a lateral flow assay or a dual path platform assay.

### LEISHMANIA FUSION POLYPEPTIDES AND USES THEREFOR

In a general aspect, the disclosure provides isolated *Leishmania* polypeptides, as described herein, including fusion polypeptides and compositions containing the same. It will be understood that while the description below relates primarily to fusion polypeptides of the present invention, the antigenic sequences covalently linked in a fusion polypeptide of the invention may also be employed in polypeptide combinations in which the antigenic sequences are not covalently linked.

Therefore, in certain embodiments, disclosed is a fusion polypeptide or polypeptide combination containing sequences derived from or related to (structurally or immunologically) the *Leishmania* sterol 24-c-methyltransferase (SMT) and non-specific nucleoside hydrolase (NH) proteins. The SMT and NH sequences used in a fusion polypeptide of the invention (or in a composition comprising a combination of separate SMT and NH polypeptides) preferably include those capable of eliciting a desired immunological response and/or capable of providing protection against Leishmaniasis in a representative *in vivo* model. In certain related embodiments, the fusion polypeptide comprises a polypeptide fragment (*e.g.,* an antigenic/immunogenic portion), multiple polypeptide fragments, or a full-length polypeptide, derived from a *Leishmania* SMT and NH proteins. The Leishmania SMT and NH polypeptides used according to the present disclosure may, in certain embodiments, be Leishmania SMT and/or NH polypeptides derived from *L. donovani, L. major* and/or *L. infantum,* or immunogenic portions or variants thereof as described herein.

As used herein, the term "polypeptide" encompasses amino acid chains of any length, including full length proteins, wherein the amino acid residues are linked by covalent bonds. A polypeptide comprising an immunogenic portion of a *Leishmania* polypeptide may consist solely of an immunogenic portion, may contain two or more immunogenic portions and/or may contain additional sequences. The additional sequences may be derived from a native *Leishmania* polypeptide or may be heterologous, and such heterologous sequences may (but need not) be immunogenic.

An "isolated polypeptide" is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. One of ordinary skill in the art would appreciate that antigenic polypeptide fragments could also be obtained from those already available in the art. Polypeptides of the invention, antigenic/immunogenic fragments thereof, and other variants may be prepared using conventional recombinant and/or synthetic techniques.

The SMT and/or NH sequences used in a fusion polypeptide or composition can be full length or substantially full length SMT and NH polypeptides, or variants thereof as described herein. Alternatively, a fusion polypeptide or composition can comprise or consist of immunogenic portions or fragments of a full length *Leishmania* SMT and/or NH polypeptide, or variants thereof.

In certain more specific embodiments, an immunogenic portion of a *Leishmania* SMT and/or NH polypeptide is a portion that is capable of eliciting an immune response (*i.e.,* cellular and/or humoral) in a presently or previously *Leishmania-infected* patient (such as a human or a dog) and/or in cultures of lymph node cells or peripheral blood mononuclear cells (PBMC) isolated from presently or previously *Leishmania*-infected individuals. The cells in which a response is elicited may comprise a mixture of cell types or may contain isolated component cells (including, but not limited to, T-cells, NK cells, macrophages, monocytes and/or B cells). In a particular embodiment, immunogenic portions of a fusion polypeptide of the invention are capable of inducing T-cell proliferation and/or a predominantly Th1-type cytokine response (*e.g.,* IL-2, IFN-γ, and/or TNF-α production by T-cells and/or NK cells, and/or IL-12 production by monocytes, macrophages and/or B cells). Immunogenic portions of the antigens described herein may generally be identified using techniques known to those of ordinary skill in the art, including the representative methods summarized in Paul, Fundamental Immunology, 5th ed., Lippincott Williams & Wilkins, 2003 and references cited therein. Such techniques include screening fusion polypeptides for the ability to react with antigen-specific antibodies, antisera and/or T cell lines or clones. As used herein, antisera and antibodies are "antigen-specific" if they specifically bind to an antigen (*i.e.,* they react with the protein in an immunoassay, and do not react detectably with unrelated proteins). Such antisera and antibodies may be prepared as described herein and using well-known techniques.

Immunogenic portions of a *Leishmania* NH and/or SMT polypeptide can be essentially any length; provided they retain one or more of the immunogenic regions of SMT and/or NH that are responsible for or contribute to the *in vivo* protection provided against leishmaniasis by one or more fusion polypeptides of the invention, as disclosed herein. In one embodiment, the ability of an immunogenic portion to react with antigen-specific antisera may be enhanced or unchanged, relative to the native protein, or may be diminished by less than 50%, and preferably less than 20%, relative to the native protein. Illustrative portions will generally be at least 10, 15, 25, 50, 150, 200, 250, 300, or 350 amino acids in length, or more, up to and including full length SMT and/or NH polypeptide.

In a particular embodiment, immunogenic portions of a *Leishmania* SMT and/or NH polypeptide are those, which when used in combination, are capable of providing protection against, for example in an *in vivo* assay as described herein, or serodiagnosis of *Leishmania* species such as *L. donovani, L. major* and/or *L. infantum,* which are believed to be causative agents of VL in humans and dogs. In addition, compositions of the invention may also be useful in blocking transmission of the causative agent of VL from dogs to humans, e.g., by reducing or eliminating the number of parasites in the blood and skin of infected dogs.

As would be recognized by the skilled artisan, a polypeptide composition of the invention may also comprise one or more polypeptides that are immunologically reactive with T cells and/or antibodies generated against a polypeptide of the invention, particularly a polypeptide having an amino acid sequence disclosed herein, or to an immunogenic fragment or variant thereof. In a specific embodiment, the polypeptide is a fusion polypeptide, as described herein.

As noted, fusion polypeptides generally comprise at least an immunogenic portion or variant of the *Leishmania* SMT and NH polypeptides described herein. In some instances, preferred immunogenic portions will be identified that have a level of immunogenic activity greater than that of the corresponding full-length polypeptide, *e.g*., having greater than about 100% or 150% or more immunogenic activity. In particular embodiments, the immunogenicity of the full-length fusion polypeptide will have additive, or greater than additive immunogenicity contributed by of each of the antigenic/immunogenic portions contained therein.

In another aspect, fusion polypeptides disclosed herein may contain multiple copies of polypeptide fragments, repeats of polypeptide fragments, or multimeric polypeptide fragments, including antigenic/immunogenic fragments, such as *Leishmania* SMT and/or NH polypeptides comprising at least about 1, 2, 3, 4, 5, 6, 7, 8, 9, 10 or more contiguous fragments of an SMT and/or NH polypeptide, in any order, and including all lengths of a polypeptide composition set forth herein, or those encoded by a polynucleotide sequence set forth herein.

In more specific embodiments, a fusion polypeptide of the invention comprises an NH amino acid sequence set forth in any one of SEQ ID NOs: 1, 3 or 5, and further comprises an SMT amino acid sequence set forth in any one of SEQ ID NOs:7, 9 or 11. Alternatively, the fusion polypeptide may comprise sequences having at least 90% or 95% or 98% identity thereto. In other more specific embodiments, the fusion polypeptide comprises, consists of, or consists essentially of the amino acid sequence set forth in SEQ ID NO: 13, or a sequence having at least 90%, 95% or 98% identity thereto.

In yet another aspect, provided are fusion polypeptides comprising one or more variants of the *Leishmania* SMT and/or NH polypeptides described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequence set forth herein, such as an NH polypeptide sequence as set forth in SEQ ID NOs: 1, 3 and 5, and/or an SMT polypeptide sequence as set forth in SEQ ID NOs: 7, 9 and 11.

In other related embodiments, a polypeptide "variant," includes polypeptides that differ from a native SMT and/or NH protein in one or more substitutions, deletions, additions and/or insertions, such that the desired immunogenicity of the variant polypeptide is not substantially diminished relative to a native SMT and/or NH polypeptide.

For example, certain variants include polypeptides that have been modified to replace one or more cysteine residues with alternative residues. Such polypeptides are referred to hereinafter as cysteine-modified polypeptides or cysteine-modified fusion polypeptides. Preferably, the modified polypeptides retain substantially the same or similar immunogenic properties as the corresponding unmodified polypeptides. In a more specific embodiment, cysteine residues are replaced with serine residues because of the similarity in the spatial arrangement of their respective side chains. However, it will be apparent to one skilled in the art that any amino acid that is incapable of interchain or intrachain disulfide bond formation can be used as a replacement for cysteine. When all or substantially all of the cysteine residues in a polypeptide or fusion polypeptide of this invention are replaced, the resulting cysteine-modified variant may be less prone to aggregation and thus easier to purify, more homogeneous, and/or obtainable in higher yields following purification.

In one embodiment, the ability of a variant to react with antigen-specific antisera may be enhanced or unchanged, relative to the native protein, or may be diminished by less than 50%, and preferably less than 20%, relative to a corresponding native or control polypeptide. In a particular embodiment, a variant of an SMT and/or NH polypeptide is one capable of providing protection, for example in an *in vivo* assay as described herein, against a *Leishmania* species such as *L. donovani, L. infantum* and/or *L. major.*

In particular embodiments, a fusion polypeptide disclosed herein comprises at least 1, at least 2, at least 3, at least 4, at least 5, at least 6, at least 7, at least 8, at least 9, or at least 10 or more substitutions, deletions, additions and/or insertions within a *Leishmania* SMT and/or NH polypeptide, where the fusion polypeptide is capable of providing protection against, for example in an *in vivo* assay as described herein, *Leishmania* species such as *L. donovani, L. major* and/or *L. infantum.*

In related embodiments, a fusion polypeptide disclosed herein comprises at least 1, at least 2, at least 3, at least 4, at least 5, at least 6, at least 7, at least 8, at least 9, or at least 10 or more substitutions, deletions, additions and/or insertions within a *Leishmania* SMT and/or NH polypeptide, where the fusion polypeptide is capable of serodiagnosis of *Leishmania* species such as *L. donovani, L. major* and/or *L. infantum.*

In many instances, a variant will contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics, *e.g*., with immunogenic characteristics. When it is desired to alter the amino acid sequence of a polypeptide to create an equivalent, or even an improved, immunogenic variant or portion of a polypeptide of the invention, one skilled in the art will typically change one or more of the codons of the encoding DNA sequence according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their biological utility or activity.

**Table 1**

| | | | | | | | | |
|---|---|---|---|---|---|---|---|---|
| Amino Acids | | Codons | | | | | | |
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982,). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i.e.* still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively on the basis of hydrophilicity.

As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide (*e.g*., poly-Histidine tag (6XHis), GST, MBP, TAP/TAG, FLAG epitope, MYC epitope, V5 epitope, VSV-G epitope, *etc.*), or to enhance binding of the polypeptide to a solid support. For example, a polypeptide may be conjugated to an immunoglobulin Fc region.

When comparing polynucleotide or polypeptide sequences, two sequences are said to be "identical" if the sequence of nucleotides or amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Alignment of sequences for comparison may be conducted using, for example, the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Natl. Acad. Sci. USA 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (*i.e.,* gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence *(i.e.,* the window size) and multiplying the results by 100 to yield the percentage of sequence identity.

Therefore, as noted above, disclosed are polynucleotide and polypeptide sequences having substantial identity to the sequences disclosed herein, for example those comprising at least 70%, 75%, 80%, 85%, 90%, 95%, 96%, 97%, 98%, or 99% or higher, sequence identity compared to a polynucleotide or polypeptide sequence of this disclosure (e.g., as set out in SEQ ID NOs: 1-14) using the methods described herein, (e.g., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like. Furthermore, it would be understood by of ordinary skill in the art that fusion polypeptides disclosed herein may comprise at least 2, at least 3, or at least 4 or more antigenic/immunogenic portions or fragments of a polypeptide comprising at least 70%, 75%, 80%, 85%, 90%, 95%, 96%, 97%, 98%, or 99% or higher, sequence identity to a *Leishmania* SMT and/or NH polypeptide that is capable of providing protection against, for example in an *in vivo* assay as described herein, or serodiagnosis of *Leishmania* species such as *L. donovani, L. major* and/or *L. infantum.*

In another aspect, fusion polypeptides are provided that comprise at least an immunogenic portion of a *Leishmania* SMT and/or NH polypeptide and further comprise a heterologous fusion partner, as well as polynucleotides encoding such fusion polypeptides. For example, a fusion polypeptide comprises one or more immunogenic portions or fragments of a *Leishmania* SMT and/or NH polypeptide and one or more additional immunogenic *Leishmania* sequences, which are joined via a peptide linkage into a single amino acid chain.

A fusion polypeptide may comprise multiple *Leishmania* antigenic epitopes wherein at least one of the epitopes is from a *Leishmania* SMT and/or NH polypeptide. As used herein an "epitope" is a portion of an antigen that reacts with blood samples from *Leishmania*-infected individuals (i.e. an epitope is specifically bound by one or more antibodies and/or T-cells present in such blood samples.

In certain embodiments, a fusion polypeptide may further comprise at least one heterologous fusion partner having a sequence that assists in providing T helper epitopes (an immunological fusion partner), preferably T helper epitopes recognized by humans, or that assists in expressing the protein (an expression enhancer) at higher yields than the native recombinant protein. Certain preferred fusion partners include both immunological and expression-enhancing fusion partners. Other fusion partners may be selected so as to increase the solubility of the protein or to enable the protein to be targeted to desired intracellular compartments. Still further fusion partners include affinity tags, such as V5, 6XHIS, MYC, FLAG, and GST, which facilitate purification of the protein. It would be understood by one having ordinary skill in the art that those unrelated sequences may, but need not, be present in a fusion polypeptide used in accordance with the present invention. Within a particular embodiment, an immunological fusion partner comprises sequence derived from protein D, a surface protein of the gram-negative bacterium *Haemophilus influenza* B (WO 91/18926). For example, one protein D derivative comprises approximately the first third of the protein (e.g., the first N-terminal 100 110 amino acids), and a protein D derivative may be lipidated. Within certain embodiments, the first 109 residues of a lipoprotein D fusion partner is included on the N-terminus to provide the polypeptide with additional exogenous T cell epitopes and to increase the expression level in *E. coli* (thus functioning as an expression enhancer). The lipid tail ensures optimal presentation of the antigen to antigen presenting cells. Other illustrative fusion partners include the non-structural protein from influenzae virus, NS1 (hemaglutinin). Typically, the N-terminal 81 amino acids are used, although different fragments that include T-helper epitopes may also be used.

In another particular embodiment, an immunological fusion partner comprises an amino acid sequence derived from the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292 (1986)). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (see Biotechnology 10:795-798 (1992)). Within a particular embodiment, a repeat portion of LYTA may be incorporated into a fusion protein. A repeat portion is found in the C-terminal region starting at residue 178. A more particular repeat portion incorporates residues 188-305.

Fusion sequences may be joined directly (*i.e.,* with no intervening amino acids) or may be joined by way of a linker sequence (*e.g*., Gly-Cys-Gly) that does not significantly diminish the immunogenic properties of the component polypeptides. The polypeptides forming the fusion protein are typically linked C-terminus to N-terminus, although they can also be linked C-terminus to C-terminus, N-terminus to N-terminus, or N-terminus to C-terminus. The polypeptides of the fusion protein can be in any order. Fusion polypeptides or fusion proteins can also include conservatively modified variants, polymorphic variants, alleles, mutants, subsequences, interspecies homologs, and immunogenic fragments of the antigens that make up the fusion protein.

Fusion polypeptides may generally be prepared using standard techniques, including recombinant technology, chemical conjugation and the like. For example, DNA sequences encoding the polypeptide components of a fusion may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in frame. This permits translation into a single fusion polypeptide that retains or in some cases exceeds the biological activity of the component polypeptides.

A peptide linker sequence may be employed to separate the fusion components by a distance sufficient to ensure that each polypeptide folds into its desired secondary and/or tertiary structures. Such a peptide linker sequence may be incorporated into the fusion polypeptide using standard techniques well known in the art. Suitable peptide linker sequences may be chosen, for example, based on one or more of the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Certain preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

In addition to recombinant fusion polypeptide expression, *Leishmania* SMT and/or NH polypeptides, immunogenic portions, variants and fusions thereof may be generated by synthetic or recombinant means. Synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (Merrifield, J. Am. Chem. Soc. 85:2149-2146, 1963). Equipment for automated synthesis of polypeptides is commercially available from suppliers such as Perkin Elmer/Applied BioSystems Division, Foster City, CA, and may be operated according to the manufacturer's instructions. Thus, for example, *Leishmania* SMT and/or NH antigens, or portions thereof, may be synthesized by this method.

Recombinant polypeptides containing portions and/or variants of a native SMT and/or NH polypeptide may be readily prepared from a DNA sequence encoding the antigen, using well known and established techniques. In particular embodiments, a fusion polypeptide comprising *Leishmania* SMT and/or NH antigens may be readily prepared from a DNA sequence encoding the cloned fused antigens. For example, supernatants from suitable host/vector systems which secrete recombinant protein into culture media may be first concentrated using a commercially available filter. Following concentration, the concentrate may be applied to a suitable purification matrix such as an affinity matrix, a size exclusion chromatography matrix or an ion exchange resin.

Alternatively, any of a variety of expression vectors known to those of ordinary skill in the art may be employed to express recombinant polypeptides. Expression may be achieved in any appropriate host cell that has been transformed or transfected with an expression vector containing a polynucleotide that encodes a recombinant polypeptide. Preferably, the host cells are *E. coli,* yeast, an insect cell line (such as *Spodoptera* or *Trichoplusia*) or a mammalian cell line, including (but not limited to) CHO, COS, HEK-293T and NS-1. The DNA sequences expressed in this manner may encode naturally occurring proteins, and fusion proteins comprising *Leishmania* antigens, such as those described herein, portions thereof, and repeats or other variants of such proteins. Expressed fusion polypeptides of this invention are generally isolated in substantially pure form. Preferably, the fusion polypeptides are isolated to a purity of at least 80% by weight, more preferably, to a purity of at least 95% by weight, and most preferably to a purity of at least 99% by weight. In general, such purification may be achieved using, for example, the standard techniques of ammonium sulfate fractionation, SDS-PAGE electrophoresis, and affinity chromatography.

*Leishmania* SMT and NH polypeptides and polynucleotides may be prepared or isolated using any of a variety of procedures and using any of a variety of *Leishmania* species including, but not limited to, *L. donovani, L. chagasi, L. infantum, L. major, L. amazonensis, L. braziliensis, L. panamensis, L. mexicana, L. tropica,* and *L. guyanensis.* Such species are available, for example, from the American Type Culture Collection (ATCC), Rockville, MD.

Regardless of the method of preparation, the SMT and NH polypeptides or fusion polypeptides produced as described above are preferably immunogenic. In certain embodiments, for example, the polypeptides (or immunogenic portions thereof) are capable of eliciting an immune response in cultures of lymph node cells and/or peripheral blood mononuclear cells (PBMC) isolated from presently or previously *Leishmania-*infected individuals. More specifically, in certain embodiments, the antigens, and immunogenic portions thereof, have the ability to induce T-cell proliferation and/or to elicit a dominantly Th1-type cytokine response (e.g., IL-2, IFN-y, and/or TNF-α production by T-cells and/or NK cells; and/or IL-12 production by monocytes, macrophages and/or B cells) in cells isolated from presently or previously *Leishmania-infected* individuals. A *Leishmania-infected* individual may be afflicted with a form of leishmaniasis (such as subclinical, cutaneous, mucosal or active visceral) or may be asymptomatic. Such individuals may be identified using methods known to those of ordinary skill in the art. Individuals with leishmaniasis may be identified based on clinical findings associated with, for example, at least one of the following: isolation of parasite from lesions, a positive skin test with *Leishmania* lysate or a positive serodiagnostic test. Asymptomatic individuals are infected individuals who have no signs or symptoms of the disease. Such individuals can be identified, for example, based on a positive serological test and/or skin test with *Leishmania* lysate.

The term "PBMC," which refers to a preparation of nucleated cells consisting primarily of lymphocytes and monocytes that are present in peripheral blood, encompasses both mixtures of cells and preparations of one or more purified cell types. PBMC may be isolated by methods known to those in the art. For example, PBMC may be isolated by density centrifugation through, for example, Ficoll™ (Winthrop Laboratories, New York). Lymph node cultures may generally be prepared by immunizing BALB/c mice (e.g., in the rear foot pad) with *Leishmania* promastigotes emulsified in complete Freünd's adjuvant. The draining lymph nodes may be excised following immunization and T-cells may be purified in an anti-mouse Ig column to remove the B cells, followed by a passage through a Sephadex G10 column to remove the macrophages. Similarly, lymph node cells may be isolated from a human following biopsy or surgical removal of a lymph node.

The ability of a fusion polypeptide to induce a response in PBMC or lymph node cell cultures may be evaluated, for example, by contacting the cells with the polypeptide and measuring a suitable response. In general, the amount of polypeptide that is sufficient for the evaluation of about 2 x 10⁵ cells ranges from about 10 ng to about 100 µg or 100 ng to about 50 µg, and preferably is about 1 µg, to 10 µg. The incubation of polypeptide (e.g., a fusion polypeptide) with cells is typically performed at 37°C for about 1-3 days. Following incubation with polypeptide, the cells are assayed for an appropriate response. If the response is a proliferative response, any of a variety of techniques well known to those of ordinary skill in the art may be employed. For example, the cells may be exposed to a pulse of radioactive thymidine and the incorporation of label into cellular DNA measured. In general, a polypeptide that results in at least a three fold increase in proliferation above background (*i.e.,* the proliferation observed for cells cultured without polypeptide) is considered to be able to induce proliferation.

Alternatively, the response to be measured may be the secretion of one or more cytokines (such as interferon-y (IFN-y), interleukin-4 (IL-4), interleukin-12 (p70 and/or p40), interleukin-2 (IL-2) and/or tumor necrosis factor-a (TNF-α)) or the change in the level of mRNA encoding one or more specific cytokines. For example, the secretion of interferon-y, interleukin-2, tumor necrosis factor-a and/or interleukin-12 is indicative of a Th1 response, which contributes to the protective effect against *Leishmania.* Assays for any of the above cytokines may generally be performed using methods known to those of ordinary skill in the art, such as an enzyme-linked immunosorbent assay (ELISA). Suitable antibodies for use in such assays may be obtained from a variety of sources such as Chemicon, Temucula, CA and PharMingen, San Diego, CA, and may generally be used according to the manufacturer's instructions. The level of mRNA encoding one or more specific cytokines may be evaluated by, for example, amplification by polymerase chain reaction (PCR). In general, a polypeptide that is able to induce, in a preparation of about 1-3 x 10⁵ cells, the production of 30 pg/mL of IL-12, IL-4, IFN-y, TNF-α or IL-12 p40, or 10 pg/mL of IL-12 p70, is considered able to stimulate production of a cytokine.

### POLYNUCLEOTIDE COMPOSITIONS

Also provided are isolated polynucleotides, particularly those encoding the polypeptide combinations and/or fusion polypeptides of the invention, as well as compositions comprising such polynucleotides. As used herein, the terms "DNA" and "polynucleotide" and "nucleic acid" refer to a DNA molecule that has been isolated free of total genomic DNA of a particular species. Therefore, a DNA segment encoding a polypeptide refers to a DNA segment that contains one or more coding sequences yet is substantially isolated away from, or purified free from, total genomic DNA of the species from which the DNA segment is obtained. Included within the terms "DNA segment" and "polynucleotide" are DNA segments and smaller fragments of such segments, and also recombinant vectors, including, for example, plasmids, cosmids, phagemids, phage, viruses, and the like.

As will be understood by those skilled in the art, the polynucleotide sequences of this invention can include genomic sequences, extra-genomic and plasmid-encoded sequences and smaller engineered gene segments that express, or may be adapted to express, proteins, fusion polypeptides, peptides and the like. Such segments may be naturally isolated, recombinant, or modified synthetically by the hand of man.

As will be recognized by the skilled artisan, polynucleotides may be single-stranded (coding or antisense) or double-stranded, and may be DNA (genomic, cDNA or synthetic) or RNA molecules. Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine. Additional coding or non-coding sequences may, but need not, be present within a polynucleotide of the present invention, and a polynucleotide may, but need not, be linked to other molecules and/or support materials.

Polynucleotides may comprise a native sequence (*i.e.,* an endogenous sequence that encodes a *Leishmania* antigen or a portion thereof) or may comprise a variant, or a biological or antigenic functional equivalent of such a sequence. In particular embodiments, polynucleotides may encode for two or more antigenic/immunogenic portions, fragments, or variants derived from the *Leishmania* SMT and/or NH antigens. In certain embodiments, polynucleotides of the present invention comprise an NH sequence as set forth in SEQ ID NOs: 2, 4 and 6, and an SMT sequence as set forth in SEQ ID NOs: 8, 10 and 12. In a related aspect, a polynucleotide as set forth in SEQ ID NO: 14 is provided, which encodes a particular NS fusion polypeptide of the present invention. Of course, portions of these sequences and variant sequences sharing identity to these sequences may also be employed (*e.g*., those having at least about 90%, 95% or 98% thereto).

Polynucleotide variants may contain one or more substitutions, additions, deletions and/or insertions, as further described below, preferably such that the immunogenicity of the encoded polypeptide is not diminished, relative to the native protein. The effect on the immunogenicity of the encoded polypeptide may generally be assessed as described herein.

For example, in certain embodiments, variants include cysteine-modified polynucleotides in which the cysteine-encoding codons are replaced with codons encoding other amino acids not capable of forming intrachain or interchain disulfide bonds. In more specific embodiments, some or all of the replacement codons encode serine because of the spatial similarity of the serine sidechain to the cysteine sidechain in the resulting polypeptide. In another specific embodiment, some or all of the replacement codons encode alanine. Illustrative methods of replacing cysteine and other codons within a polynucleotide are well known (e.g., U.S. Patent No. 4,816,566, and Proc Natl Acad Sci 97 (15): 8530, 2000).

The term "variants" also encompasses homologous genes of xenogenic origin.

In additional embodiments, isolated polynucleotides disclosed herein comprise various lengths of contiguous stretches of sequence identical to or complementary to SMT and NH, such as those sequences disclosed herein. For example, polynucleotides are provided that comprise at least about 15, 20, 30, 40, 50, 75, 100, 150, 200, 300, 400, 500 or 1000 or more contiguous nucleotides of two or more of the sequences disclosed herein as well as all intermediate lengths there between. It will be readily understood that "intermediate lengths", in this context, means any length between the quoted values, such as 16, 17, 18, 19, *etc.*; 21, 22, 23, *etc.*; 30, 31, 32, *etc.*; 50, 51, 52, 53, *etc.*; 100, 101, 102, 103, *etc.*; 150, 151, 152, 153, *etc.*; including all integers through 200-500; 500-1,000, and the like.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a polynucleotide fragment of almost any length may be employed; with the total length preferably being limited by the ease of preparation and use in the intended recombinant DNA protocol.

Moreover, it will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention, for example polynucleotides that are optimized for human and/or primate codon selection. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

*Leishmania* polynucleotides and fusions thereof may be prepared, manipulated and/or expressed using any of a variety of well established techniques known and available in the art. In particular embodiments, fusions comprise two or more polynucleotide sequences encoding *Leishmania* SMT and NH antigens.

For example, polynucleotide sequences or fragments thereof which encode polypeptides of the invention, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide of the present invention.

As will be understood by those of skill in the art, it may be advantageous in some instances to produce fusion polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover polynucleotide sequences can be engineered using methods generally known in the art in order to alter fusion polypeptide encoding sequences for a variety of reasons, including but not limited to, alterations which modify the cloning, processing, expression and/or immunogenicity of the gene product.

In order to express a desired fusion polypeptide comprising two or more antigenic/immunogenic fragments or portions of SMT and/or NH polypeptides, a nucleotide sequence encoding the fusion polypeptide, or a functional equivalent, may be inserted into appropriate expression vector, *i.e.,* a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described in Sambrook et al., Molecular Cloning, A Laboratory Manual (2001), and Ausubel et al., Current Protocols in Molecular Biology (January 2008, updated edition).

A variety of expression vector/host systems are known and may be utilized to contain and express polynucleotide sequences. These include, but are not limited to, microorganisms such as bacteria transformed with recombinant bacteriophage, plasmid, or cosmid DNA expression vectors; yeast (such as *Saccharomyces* or *Pichia*) transformed with yeast expression vectors; insect cell systems infected with virus expression vectors (*e.g.,* baculovirus); plant cell systems transformed with virus expression vectors (*e.g*., cauliflower mosaic virus, CaMV; tobacco mosaic virus, TMV) or with bacterial expression vectors (*e.g*., Ti or pBR322 plasmids); or animal cell systems.

The "control elements" or "regulatory sequences" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, Md.) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, a number of expression vectors may be selected depending upon the use intended for the expressed polypeptide. For example, when large quantities are needed, vectors which direct high level expression of fusion proteins that are readily purified may be used. Such vectors include, but are not limited to, the multifunctional *E. coli* cloning and expression vectors such as PBLUESCRIPT (Stratagene), in which the sequence encoding the polypeptide of interest may be ligated into the vector in frame with sequences for the amino-terminal Met and the subsequent 7 residues of β-galactosidase so that a hybrid protein is produced; pIN vectors (Van Heeke & Schuster, J. Biol. Chem. 264:5503 5509 (1989)); and the like. pGEX Vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, *Saccharomyces cerevisiae,* a number of vectors containing constitutive or inducible promoters such as alpha factor, alcohol oxidase, and PGH may be used. For reviews, see Ausubel *et al*. (supra) and Grant et al., Methods Enzymol. 153:516-544 (1987).

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, EMBO J. 6:307-311 (1987)). Alternatively, plant promoters such as the small subunit of RUBISCO or heat shock promoters may be used (Coruzzi et al., EMBO J. 3:1671-1680 (1984); Broglie et al., Science 224:838-843 (1984); and Winter et al., Results Probl. Cell Differ. 17:85-105 (1991)). These constructs can be introduced into plant cells by direct DNA transformation or pathogen-mediated transfection. Such techniques are described in a number of generally available reviews (see, *e.g*., Hobbs in McGraw Hill, Yearbook of Science and Technology, pp. 191-196 (1992)).

An insect system may also be used to express a polypeptide of interest. For example, in one such system, *Autographa californica* nuclear polyhedrosis virus (AcNPV) is used as a vector to express foreign genes in *Spodoptera frugiperda* cells or in *Trichoplusia* larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, *S*. *frugiperda* cells or *Trichoplusia* larvae in which the polypeptide of interest may be expressed (Engelhard et al., Proc. Natl. Acad. Sci. U.S.A. 91:3224-3227 (1994)).

In mammalian host cells, a number of viral-based expression systems are generally available. For example, in cases where an adenovirus is used as an expression vector, sequences encoding a polypeptide of the present invention may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan & Shenk, Proc. Natl. Acad. Sci. U.S.A. 81:3655-3659 (1984)). In addition, transcription enhancers, such as the Rous sarcoma virus (RSV) enhancer, may be used to increase expression in mammalian host cells.

Specific initiation signals may also be used to achieve more efficient translation of sequences encoding a fusion polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf. et al., Results Probl. Cell Differ. 20:125-162 (1994)).

In addition, a host cell strain may be chosen for its ability to modulate the expression of the inserted sequences or to process the expressed fusion protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, HeLa, MDCK, HEK293, and W138, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a fusion polynucleotide of the present invention may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus *thymidine kinase* (Wigler et al., Cell 11:223-232 (1977)) and *adenine phosphoribosyltransferase* (Lowy et al., Cell 22:817-823 (1990)) genes which can be employed in *tk-* or *aprt-* cells, respectively. Also, antimetabolite, antibiotic or herbicide resistance can be used as the basis for selection; for example, dhfr which confers resistance to methotrexate (Wigler et al., Proc. Natl. Acad. Sci. U.S.A. 77:3567-70 (1980)); *npt,* which confers resistance to the aminoglycosides, neomycin and G-418 (Colbere-Garapin et al., J. Mol. Biol. 150:1-14 (1981)); and *als* or *pat,* which confer resistance to chlorsulfuron and phosphinotricin acetyltransferase, respectively (Murry, supra). Additional selectable genes have been described, for example, *trpB,* which allows cells to utilize indole in place of tryptophan, or *hisD,* which allows cells to utilize histinol in place of histidine (Hartman & Mulligan, Proc. Natl. Acad. Sci. U.S.A. 85:8047-51 (1988)). The use of visible markers has gained popularity with such markers as anthocyanins, β-glucuronidase and its substrate GUS, and luciferase and its substrate luciferin, being widely used not only to identify transformants, but also to quantify the amount of transient or stable protein expression attributable to a specific vector system (Rhodes et al., Methods Mol. Biol. 55:121-131 (1995)).

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). These and other assays are described, among other places, in Hampton et al., Serological Methods, a Laboratory Manual (1990) and Maddox et al., J. Exp. Med. 158:1211-1216 (1983).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any portions thereof may be cloned into a vector for the production of an mRNA probe. Such vectors are known in the art, are commercially available, and may be used to synthesize RNA probes *in vitro* by addition of an appropriate RNA polymerase such as T7, T3, or SP6 and labeled nucleotides. These procedures may be conducted using a variety of commercially available kits. Suitable reporter molecules or labels, which may be used, include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides of the invention may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins.
In addition to recombinant production methods, fusion polypeptides of the invention, and fragments thereof, may be produced by direct peptide synthesis using solid-phase techniques (Merrifield, J. Am. Chem. Soc. 85:2149-2154 (1963)). Protein synthesis may be performed using manual techniques or by automation. Automated synthesis may be achieved, for example, using Applied Biosystems 431A Peptide Synthesizer (Perkin Elmer). Alternatively, various fragments, for example, immunogenic fragments from *Leishmania* SMT and NH antigens, may be chemically synthesized separately and combined using chemical methods to produce the full length molecule.

### PHARMACEUTICAL AND VACCINE COMPOSITIONS

In certain aspects, the polypeptides, polynucleotides, portions, variants, fusion polypeptides, *etc.,* as described herein, are incorporated into pharmaceutical compositions or vaccines. Pharmaceutical compositions generally comprise one or more polypeptides, polynucleotides, portions, variants, fusion polypeptides, *etc.,* as described herein, in combination with a physiologically acceptable carrier. Vaccines, also referred to as immunogenic compositions, generally comprise one or more of the polypeptides, polynucleotides, portions, variants, fusion proteins, *etc.,* as described herein, in combination with an immunostimulant, such as an adjuvant. In particular embodiments, the pharmaceutical compositions comprise fusion polypeptides containing *Leishmania* SMT and NH polypeptide antigens (or portions or variants thereof) that are capable of providing protection against, for example in an *in vivo* assay as described herein, *Leishmania* species such as *L. donovani, L. major* and/or *L. infantum.*

An immunostimulant may be any substance that enhances or potentiates an immune response (antibody and/or cell-mediated) to an exogenous antigen. Examples of immunostimulants include adjuvants, biodegradable microspheres (*e.g*., polylactic galactide) and liposomes (into which the compound is incorporated; see, *e.g.,* Fullerton, U.S. Pat. No. 4,235,877). Vaccine preparation is generally described in, for example, Powell & Newman, eds., Vaccine Design (the subunit and adjuvant approach) (1995).

Any of a variety of immunostimulants may be employed in the vaccines disclosed herein. For example, an adjuvant may be included. Many adjuvants contain a substance designed to protect the antigen from rapid catabolism, such as aluminum hydroxide or mineral oil, and a stimulator of immune responses, such as lipid A (natural or synthetic), *Bordatella pertussis* or *Mycobacterium* species or *Mycobacterium*-derived proteins. Suitable adjuvants are commercially available as, for example, Freund's Incomplete Adjuvant and Complete Adjuvant (Difco Laboratories, Detroit, Mich.); Merck Adjuvant 65 (Merck and Company, Inc., Rahway, N.J.); AS-2 and derivatives thereof (GlaxoSmithKline Beecham, Philadelphia, Pa.); CWS, TDM, LeIF, aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Also disclosed are vaccine and pharmaceutical compositions that include one or more toll-like receptor agonists (TLR agonist). In more specific embodiments, for example, the compositions of the invention include Toll-like receptor agonists, such as TLR7 agonists and TLR7/8 agonists. In certain embodiments the TLR agonist is capable of delivering a biological signal by interacting with at least one TLR that is selected from TLR-2, TLR-3, TLR-4, TLR-5, TLR-6, TLR-7, TLR-8 and TLR-9.

Toll-like receptors (TLR) include cell surface transmembrane receptors of the innate immune system that confer early-phase recognition capability to host cells for a variety of conserved microbial molecular structures such as may be present in or on a large number of infectious pathogens. (*e*.*g*., Armant et al., 2002 Genome Biol. 3(8):reviews3011.1-3011.6; Fearon et al., 1996 Science 272:50; Medzhitov et al., 1997 Curr. Opin. Immunol. 9:4; Luster 2002 Curr. Opin. Immunol. 14:129; Lien et al. 2003 Nat. Immunol. 4:1162; Medzhitov, 2001 Nat. Rev. Immunol. 1:135; Takeda et al., 2003 Ann Rev Immunol. 21:335; Takeda et al. 2005 Int. Immunol. 17:1; Kaisho et al., 2004 Microbes Infect. 6:1388; Datta et al., 2003 J. Immunol. 170:4102).

Induction of TLR-mediated signal transduction to potentiate the initiation of immune responses via the innate immune system may be effected by TLR agonists, which engage cell surface TLR or cytoplasmic TLR. For example, lipopolysaccharide (LPS) may be a TLR agonist through TLR2 or TLR4 (Tsan et al., 2004 J. Leuk. Biol. 76:514; Tsan et al., 2004 Am. J. Physiol. Cell Phsiol. 286:C739; Lin et al., 2005 Shock 24:206); poly(inosine-cytidine) (polyl:C) may be a TLR agonist through TLR3 (Salem et al., 2006 Vaccine 24:5119); CpG sequences (oligodeoxynucleotides containing unmethylated cytosine-guanosine or "CpG" dinucleotide motifs, e.g., CpG 7909, Cooper et al., 2005 AIDS 19:1473; CpG 10101 Bayes et al. Methods Find Exp Clin Pharmacol 27:193; Vollmer et al. Expert Opinion on Biological Therapy 5:673; Vollmer et al., 2004 Antimicrob. Agents Chemother. 48:2314; Deng et al., 2004 J. Immunol. 173:5148) may be TLR agonists through TLR9 (Andaloussi et a., 2006 Glia 54:526; Chen et al., 2006 J. Immunol. 177:2373); peptidoglycans may be TLR2 and/or TLR6 agonists (Soboll et al., 2006 Biol. Reprod. 75:131; Nakao et al., 2005 J. Immunol. 174:1566); 3M003 (4-amino-2-(ethoxymethyl)-α,α-dimethyl-6,7,8,9-tetrahydro-1H-imidazo[4,5-c]quinoline-1-ethanol hydrate, Mol. Wt. 318 Da from 3M Pharmaceuticals, St. Paul, MN, which is also a source of the related compounds 3M001 and 3M002; Gorden et al., 2005 J. Immunol. 174:1259) may be a TLR7 agonist (Johansen 2005 Clin. Exp. Allerg. 35:1591) and/or a TLR8 agonist (Johansen 2005); flagellin may be a TLR5 agonist (Feuillet et al., 2006 Proc. Nat. Acad. Sci. USA 103:12487); and hepatitis C antigens may act as TLR agonists through TLR7 and/or TLR9 (Lee et al., 2006 Proc. Nat. Acad. Sci. USA 103:1828; Horsmans et al., 2005 Hepatol. 42:724). Other TLR agonists are known *(e.g.,* Schirmbeck et al., 2003 J. Immunol. 171:5198) and may be used according to certain of the presently described embodiments.

For example, and by way of background (see, *e.g*., U.S. Patent No. 6,544,518) immunostimulatory oligonucleotides containing ummethylated CpG dinucleotides ("CpG") are known as being adjuvants when administered by both systemic and mucosal routes (WO 96/02555, EP 468520, Davis et al., J. Immunol, 1998. 160(2):870-876; McCluskie and Davis, J. Immunol., 1998, 161(9):4463-6). CpG is an abbreviation for cytosine-guanosine dinucleotide motifs present in DNA. The central role of the CG motif in immunostimulation was elucidated by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the dinucleotide CG motif is not methylated, but other unmethylated CpG sequences are known to be immunostimulatory and may be used in certain embodiments of the present invention. CpG when formulated into vaccines, may be administered in free solution together with free antigen (WO 96/02555; McCluskie and Davis, *supra*) or covalently conjugated to an antigen (PCT Publication No. WO 98/16247), or formulated with a carrier such as aluminium hydroxide (*e.g.,* Davis et al. *supra,* Brazolot-Millan et al., Proc.Natl.Acad.Sci., USA, 1998, 95(26), 15553-8).

Other illustrative oligonucleotides for use in compositions present invention will often contain two or more dinucleotide CpG motifs separated by at least three, more preferably at least six or more nucleotides. The oligonucleotides of the present invention are typically deoxynucleotides. In one embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in U.S. Pat. Nos. 5,666,153, 5,278,302 and WO95/26204.

Other examples of oligonucleotides have sequences that are disclosed in the following publications; for certain herein disclosed embodiments the sequences preferably contain phosphorothioate modified internucleotide linkages:
CPG 7909: Cooper et al., "CPG 7909 adjuvant improves hepatitis B virus vaccine seroprotection in antiretroviral-treated HIV-infected adults." AIDS, 2005 Sep 23;19(14):1473-9.

CpG 10101: Bayes et al., "Gateways to clinical trials." Methods Find. Exp. Clin. Pharmacol. 2005 Apr;27(3):193-219.

Vollmer J., "Progress in drug development of immunostimula-tory CpG oligodeoxynucleotide ligands for TLR9." Expert Opinion on Biological Therapy. 2005 May; 5(5): 673-682

Alternative CpG oligonucleotides may comprise variants of the preferred sequences described in the above-cited publications that differ in that they have inconsequential nucleotide sequence substitutions, insertions, deletions and/or additions thereto. The CpG oligonucleotides utilized in certain embodiments of the present invention may be synthesized by any method known in the art (*e.g*., EP 468520). Conveniently, such oligonucleotides may be synthesized utilising an automated synthesizer. The oligonucleotides are typically deoxynucleotides. In a preferred embodiment the internucleotide bond in the oligonucleotide is phosphorodithioate, or more preferably phosphorothioate bond, although phosphodiesters are also within the scope of the presently contemplated embdiments. Oligonucleotides comprising different internucleotide linkages are also contemplated, *e.g*., mixed phosphorothioate phophodiesters. Other internucleotide bonds which stabilize the oligonucleotide may also be used.

In certain more specific embodiments the TLR agonist is selected from lipopolysaccharide, peptidoglycan, polyl:C, CpG, 3M003, flagellin, *Leishmania* homolog of eukaryotic ribosomal elongation and initiation factor 4a (LeIF) and at least one hepatitis C antigen.

Still other illustrative adjuvants include imiquimod, gardiquimod and resiquimod (all available from Invivogen), and related compounds, which are known to act as TLR7/8 agonists. A compendium of adjuvants that may be useful in vaccines is provided by Vogel et al., Pharm Biotechnol 6:141 (1995).

Compositions of the invention may also employ adjuvant systems designed to induce an immune response predominantly of the Th1 type. High levels of Th1-type cytokines (*e.g*., IFN-γ, TNF-α., IL-2 and IL-12) tend to favor the induction of cell mediated immune responses to an administered antigen. In contrast, high levels of Th2-type cytokines (*e.g.,* IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly of the Th1-type, the level of Th1-type cytokines will increase to a greater extent than the level of Th2-type cytokines. The levels of these cytokines may be readily assessed using standard assays. For a review of the families of cytokines, see Mossman & Coffman, Ann. Rev. Immunol. 7:145-173 (1989).

Certain adjuvants for use in eliciting a predominantly Th1-type response include, for example, a combination of monophosphoryl lipid A, preferably 3-de-O-acylated monophosphoryl lipid A (3D-MPL™), together with an aluminum salt (U.S. Pat. Nos. 4,436,727; 4,877,611; 4,866,034; and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Pat. Nos. 6,008,200 and 5,856,462. Immunostimulatory DNA sequences are also described, for example, by Sato et al., Science 273:352 (1996). Another illustrative adjuvant comprises a saponin, such as Quil A, or derivatives thereof, including QS21 and QS7 (Aquila Biopharmaceuticals Inc., Framingham, Mass.); Escin; Digitonin; or Gypsophila or Chenopodium quinoa saponins. Other illustrative formulations include more than one saponin in the adjuvant combinations of the present invention, for example combinations of at least two of the following group comprising QS21, QS7, Quil A, β- escin, or digitonin.

In a particular embodiment, the adjuvant system includes the combination of a monophosphoryl lipid A and a saponin derivative, such as the combination of QS21 and 3D-MPL™ adjuvant, as described in WO 94/00153, or a less reactogenic composition where the QS21 is quenched with cholesterol, as described in WO 96/33739. Other formulations comprise an oil-in-water emulsion and tocopherol. Another adjuvant formulation employing QS21, 3D-MPL™ adjuvant and tocopherol in an oil-in-water emulsion is described in WO 95/17210.

In certain preferred embodiments, the adjuvant used in the present invention is a glucopyranosyl lipid A (GLA) adjuvant, as described in U.S. Patent Application Publication No. 20080131466. For example, in one embodiment, the GLA adjuvant used in the context of the present invention has the following structure: 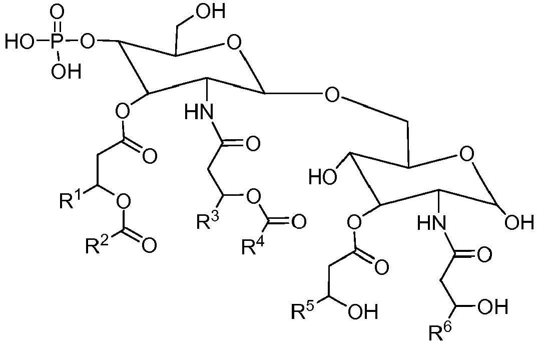 where: R¹, R³, R⁵ and R⁶ are C₁₁-C₂₀ alkyl; and R² and R⁴ are C₁₂-C₂₀ alkyl.

In a more specific embodiment, the GLA has the formula set forth above wherein R¹, R³, R⁵ and R⁶ are C₁₁₋₁₄ alkyl; and R² and R⁴ are C₁₂₋₁₅ alkyl.

In a more specific embodiment, the GLA has the formula set forth above wherein R¹, R³, R⁵ and R⁶ are C₁₁ alkyl; and R² and R⁴ are C₁₃ alkyl.

Another enhanced adjuvant system involves the combination of a CpG-containing oligonucleotide and a saponin derivative as disclosed in WO 00/09159.

Other illustrative adjuvants include Montanide ISA 720 (Seppic, France), SAF (Chiron, Calif., United States), ISCOMS (CSL), MF-59 (Chiron), the SBAS series of adjuvants (*e.g*., SBAS-2, AS2', AS2," SBAS-4, or SBAS6, available from SmithKline Beecham, Rixensart, Belgium), Detox, RC-529 (Corixa, Hamilton, Mont.) and other aminoalkyl glucosaminide 4-phosphates (AGPs), such as those described in pending U.S. patent application Ser. Nos. 08/853,826 and 09/074,720, and polyoxyethylene ether adjuvants such as those described in WO 99/52549A1.

The vaccine and pharmaceutical compositions may be formulated using any of a variety of well known procedures. In certain embodiments, the vaccine or pharmaceutical compositions are prepared as stable emulsions (e.g., oil-in-water emulsions) or as aqueous solutions.

Compositions of the invention may also, or alternatively, comprise T cells specific for fusion polypeptide comprising immunogenic/antigenic portions or fragments of *Leishmania* SMT and NH antigens or variants thereof, described herein. Such cells may generally be prepared *in vitro* or *ex vivo,* using standard procedures. For example, T cells may be isolated from bone marrow, peripheral blood, or a fraction of bone marrow or peripheral blood of a patient. Alternatively, T cells may be derived from related or unrelated humans, non-human mammals, cell lines or cultures.

T cells may be stimulated with a fusion polypeptide comprising *Leishmania* SMT and NH antigens or immunogenic portions or variants thereof, polynucleotide encoding such a fusion polypeptide, and/or an antigen presenting cell (APC) that expresses such a fusion polypeptide. Such stimulation is performed under conditions and for a time sufficient to permit the generation of T cells that are specific for the polypeptide. In certain embodiments, the polypeptide or polynucleotide is present within a delivery vehicle, such as a microsphere, to facilitate the generation of specific T cells.

T cells are considered to be specific for a fusion polypeptide of the invention if the T cells specifically proliferate, secrete cytokines or kill target cells coated with the fusion polypeptide or expressing a gene encoding the fusion polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070 (1994)). Alternatively, detection of the proliferation of T cells may be accomplished by a variety of known techniques. For example, T cell proliferation can be detected by measuring an increased rate of DNA synthesis (*e.g*., by pulse-labeling cultures of T cells with tritiated thymidine and measuring the amount of tritiated thymidine incorporated into DNA). Contact with a polypeptide of the invention (100ng/ml-100ng/ml, preferably 200ng/ml-25µg/ml) for 3-7 days should result in at least a two fold increase in proliferation of the T cells. Contact as described above for 2-3 hours should result in activation of the T cells, as measured using standard cytokine assays in which a two fold increase in the level of cytokine release (*e.g*., TNF or IFN-γ) is indicative of T cell activation (see Coligan et al., Current Protocols in Immunology, vol. 1 (1998)). T cells that have been activated in response to a polypeptide, polynucleotide or polypeptide-expressing APC may be CD4+ and/or CD8+. Protein-specific T cells may be expanded using standard techniques. Within preferred embodiments, the T cells are derived from a patient, a related donor or an unrelated donor, and are administered to the patient following stimulation and expansion.

In the compositions of the invention, formulation of pharmaceutically-acceptable excipients and carrier solutions is well-known to those of skill in the art, as is the development of suitable dosing and treatment regimens for using the particular compositions described herein in a variety of treatment regimens, including *e.g*., oral, parenteral, intravenous, intranasal, intradermal, subcutaneous and intramuscular administration and formulation.

In certain applications, the compositions disclosed herein may be delivered via oral administration to a subject. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

In certain circumstances it will be desirable to deliver the compositions disclosed herein parenterally, intravenously, intramuscularly, or even intraperitoneally as described, for example, in U.S. Pat. No. 5,543,158; U.S. Pat. No. 5,641,515 and U.S. Pat. No. 5,399,363. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions (U.S. Pat. No. 5,466,468, In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion (see, *e.g.,* Remington's Pharmaceutical Sciences, 15th Edition, pp. 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, and the general safety and purity standards as required by FDA Office of Biologics standards.

Sterile injectable solutions are prepared by incorporating the active compounds in the required amount in the appropriate solvent with the various other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective for treatment of leishmaniasis. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known to one of ordinary skill in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood to one of ordinary skill in the art. Typically, such compositions are prepared as injectables, either as liquid solutions or suspensions; solid forms suitable for solution in, or suspension in, liquid prior to injection can also be prepared. The preparation can also be emulsified.

In certain embodiments, the compositions of the present invention may be delivered by intranasal sprays, inhalation, and/or other aerosol delivery vehicles. Methods for delivering genes, polynucleotides, and peptide compositions directly to the lungs via nasal aerosol sprays has been described *e.g.,* in U.S. Pat. No. 5,756,353 and U.S. Pat. No. 5,804,212. Likewise, the delivery of drugs using intranasal microparticle resins (Takenaga *et al*., 1998) and lysophosphatidyl-glycerol compounds (U.S. Pat. No. 5,725,871, are also well-known in the pharmaceutical arts. Likewise, transmucosal drug delivery in the form of a polytetrafluoroetheylene support matrix is described in U.S. Pat. No. 5,780,045.

In certain embodiments, the delivery may occur by use of liposomes, nanocapsules, microparticles, microspheres, lipid particles, vesicles, and the like, for the introduction of compositions comprising a fusion polypeptide as describe herein into suitable host cells. In particular, the compositions of the present invention may be formulated for delivery either encapsulated in a lipid particle, a liposome, a vesicle, a nanosphere, a nanoparticle or the like. The formulation and use of such delivery vehicles can be carried out using known and conventional techniques.

A pharmaceutical or immunogenic composition may, alternatively, contain an immunostimulant and a DNA molecule encoding one or more of the polypeptides or fusion polypeptides as described above, such that a desired polypeptide is generated *in situ.* In such compositions, the DNA encoding the fusion protein may be present within any of a variety of delivery systems known to those of ordinary skill in the art, including nucleic acid expression systems, bacteria and viral expression systems. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface. In a particular embodiment, the DNA may be introduced using a viral expression system (*e.g*., vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749 (1993) and reviewed by Cohen, Science 259:1691-1692 (1993). The uptake of naked DNA may be increased by coating the DNA onto biodegradable beads, which are efficiently transported into the cells.

The pharmaceutical compositions and vaccines disclosed herein may be used, in certain embodiments, to induce protective immunity against *Leishmania* species such as *L. donovani, L. major* and/or *L. infantum* in a patient, such as a human or a dog, to prevent leishmaniasis or diminish its severity. The compositions and vaccines may also be used to stimulate an immune response, which may be cellular and/or humoral, in a patient, for treating an individual already infected. In one embodiment, for *Leishmania-*infected patients, the immune responses generated include a preferential Th1 immune response (*i.e.,* a response characterized by the production of the cytokines interleukin-1, interleukin-2, interleukin-12 and/or interferon-y, as well as tumor necrosis factor-a). In another embodiment, for uninfected patients, the immune response involves production of interleukin-12 and/or interleukin-2, or the stimulation of gamma delta T-cells. In either category of patient, the response stimulated may include IL-12 production. Such responses may also be elicited in biological samples of PBMC or components thereof derived from *Leishmania*-infected or uninfected individuals. As noted above, assays for any of the above cytokines, as well as other known cytokines, may generally be performed using methods known to those of ordinary skill in the art, such as an enzyme-linked immunosorbent assay (ELISA).

Appropriate doses and methods of fusion polypeptide administration for these purposes can be readily determined by a skilled artisan using available knowledge in the art and/or routine techniques. Routes and frequency of administration, as well as dosage, for the above aspects of the present invention may vary from individual to individual and may parallel those currently being used in immunization against other infections, including protozoan, viral and bacterial infections. For example, in one embodiment, between 1 and 12 doses of composition having a fusion polypeptide, which comprises *Leishmania* SMT and NH polypeptides or immunogenic/antigenic portions, fragments or variants thereof, are administered over a 1 year period. Booster vaccinations may be given periodically thereafter as needed or desired. Of course, alternate protocols may be appropriate for individual patients. In a particular embodiment, a suitable dose is an amount of fusion polypeptide or DNA encoding such a peptide that, when administered as described above, is capable of eliciting an immune response in an immunized patient sufficient to protect the patient from leishmaniasis caused by *Leishmania* species such as *L*. *donovani, L. major* and/or *L. infantum* for at least 1-2 years. In general, the amount of fusion polypeptide present in a dose (or produced *in situ* by the DNA in a dose) ranges from about 100 ng to about 1mg per kg of host, typically from about 10 µg to about 100 µg. Suitable dose sizes will vary with the size of the patient, but will typically range from about 0.1 mL to about 5 mL.

### DIAGNOSTIC COMPOSITIONS, METHODS AND KITS

In another aspect, this invention provides compounds and methods for detecting leishmaniasis in individuals and in blood supplies. In particular embodiments, the individual is a mammal. In more particular embodiments, the mammal is a human or canine.

For example, the fusion polypeptides and polynucleotides of the can be used as effective diagnostic reagents for detecting and/or monitoring *Leishmania* infection in a patient. For example, the compositions, fusion polypeptides, and polynucleotides of the invention may be used in *in vitro* and *in vivo* assays for detecting humoral antibodies or cell-mediated immunity against *Leishmania* for diagnosis of infection, monitoring of disease progression or test-of-cure evaluation. In particular embodiments, the fusion polypeptides and polynucleotides are useful diagnostic reagents for serodiagnosis and whole blood assay in patients having leishmaniasis caused by *Leishmania* species such as *L. donovani, L. major* and/or *L. infantum.*

In one aspect, the diagnostic methods and kits preferably employ a fusion polypeptide or polypeptide combination as described herein, such as a fusion polypeptide comprising SMT and/or NH polypeptide fragments, repeats of polypeptide fragments, or multimeric polypeptide fragments, including antigenic/immunogenic fragments. In another more specific aspect, fusion polypeptides of the present invention may comprise two or more *Leishmania* antigen fragments wherein at least one fragment is from an NH amino acid sequence set forth in SEQ ID NOs: 1, 3 or 5, and at least one fragment is from an SMT amino acid sequence set forth in SEQ ID NOs: 7, 9 or 11. In a more particular embodiment, an illustrative fusion polypeptide comprises the amino acid sequence set forth in SEQ ID NO: 13. In another embodiment, the diagnostic methods and kits preferably employ a fusion polypeptide comprising at least 1, at least 2, at least 3, or at least 4 immunogenic/antigenic portions or fragments of *Leishmania* SMT and NH polypeptides, variants or the like, optionally in combination with one or more other *Leishmania* antigens or non-Leishmania sequences, as described herein or obtainable in the art.

The antigens may be used in essentially any assay format desired, e.g., as individual antigens assayed separately, as multiple antigens assays simultaneously (e.g., a fusion polypeptide), as antigens immobilized on a solid support such as an array, or the like.

In one embodiment, there are provided diagnostic kits for detecting *Leishmania* infection in a biological sample, comprising (a) a fusion polypeptide comprising *Leishmania* SMT and NH polypeptides or variants thereof as described herein, and (b) a detection reagent.

In another embodiment, there are provided diagnostic kits for detecting *Leishmania* infection in a biological sample, comprising (a) antibodies or antigen binding fragments thereof that are specific for a fusion polypeptide comprising *Leishmania* SMT and NH polypeptides or variants thereof as described herein, and (b) a detection reagent.

In another embodiment, methods are provided for detecting the presence of *Leishmania* infection in a biological sample, comprising (a) contacting a biological sample with a fusion polypeptide comprising *Leishmania* SMT and NH polypeptides or variants thereof described herein; and (b) detecting in the biological sample the presence of antibodies that bind to the fusion polypeptide.

In another embodiment, methods are provided for detecting the presence of *Leishmania* infection in a biological sample, comprising (a) contacting a biological sample with at least 2 monoclonal antibodies that bind to a fusion polypeptide comprising *Leishmania* SMT and NH polypeptides or variants thereof described herein; and (b) detecting in the biological sample the presence of *Leishmania* proteins that bind to the monoclonal antibody.

One of ordinary skill in the art would recognize that the methods and kits described herein may be used to detect all types of leishmaniasis, depending on the particular combination of immunogenic portions of *Leishmania* antigens present in the fusion polypeptide.

There are a variety of assay formats known to those of ordinary skill in the art for using a fusion polypeptide to detect antibodies in a sample. *See, e.g.,* Harlow and Lane, Antibodies. A Laboratory Manual, Cold Spring Harbor Laboratory Press, 1988. In one embodiment, the assay involves the use of fusion polypeptide immobilized on a solid support to bind to and remove the antibody from the sample. The bound antibody may then be detected using a detection reagent that binds to the antibody/peptide complex and contains a detectable reporter group. Suitable detection reagents are well known and include, for example, antibodies that bind to the antibody/polypeptide complex and free polypeptide labeled with a reporter group (*e.g*., in a semi-competitive assay). Suitable reporter groups are also well known and include, for example, fluorescent labels, enzyme labels, radioisotopes, chemiluminescent labels, electrochemiluminescent labels, bioluminescent labels, polymers, polymer particles, metal particles, haptens, and dyes. Alternatively, a competitive assay may be utilized, in which an antibody that binds to a fusion polypeptide of the present invention labeled with a reporter group and allowed to bind to the immobilized fusion polypeptide after incubation of the fusion polypeptide with the sample. The extent to which components of the sample inhibit the binding of the labeled antibody to the fusion polypeptide is indicative of the reactivity of the sample with the immobilized fusion polypeptide.

The solid support may be any material known to those of ordinary skill in the art to which the fusion polypeptide may be attached. For example, the support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Pat. No. 5,359,681.

The fusion polypeptide may be bound to the solid support using a variety of techniques known to those in the art, which are amply described in the patent and scientific literature. In the context of the present invention, the term "bound" refers to both non-covalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. In such cases, adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of fusion polypeptide ranging from about 10 ng to about 1 µg, and preferably about 100 ng, is sufficient to bind an adequate amount of antigen. Nitrocellulose will bind approximately 100 µg of protein per cm³.

Covalent attachment of fusion polypeptide to a solid support may generally be achieved by first reacting the support with a bifunctional reagent that will react with both the support and a functional group, such as a hydroxyl or amino group, on the fusion polypeptide. For example, the fusion polypeptide may be bound to a support having an appropriate polymer coating using benzoquinone or by condensation of an aldehyde group on the support with an amine and an active hydrogen on the polypeptide (*see, e.g.,* Pierce Immunotechnology Catalog and Handbook (1991) at A12-A13).

In certain embodiments, the assay is an enzyme linked immunosorbent assay (ELISA). This assay may be performed by first contacting a fusion polypeptide of the present invention that has been immobilized on a solid support, commonly the well of a microtiter plate, with the sample, such that antibodies to the *Leishmania* antigens of the fusion polypeptide within the sample are allowed to bind to the immobilized fusion polypeptide. Unbound sample is then removed from the immobilized fusion polypeptide and a detection reagent capable of binding to the immobilized antibody-polypeptide complex is added. The amount of detection reagent that remains bound to the solid support is then determined using a method appropriate for the specific detection reagent.

Once the fusion polypeptide is immobilized on the support, the remaining protein binding sites on the support are typically blocked. Any suitable blocking agent known to those of ordinary skill in the art, such as bovine serum albumin (BSA) or Tween 20™ (Sigma Chemical Co., St. Louis, Mo.) may be employed. The immobilized polypeptide is then incubated with the sample, and antibody (if present in the sample) is allowed to bind to the antigen. The sample may be diluted with a suitable diluent, such as phosphate-buffered saline (PBS) prior to incubation. In general, an appropriate contact time (*i.e.*, incubation time) is that period of time that is sufficient to permit detection of the presence of antibody within a *Leishmania*-infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (such as, for example, Protein A, Protein G, immunoglobulin, lectin or free antigen) conjugated to a reporter group. Preferred reporter groups include enzymes (such as horseradish peroxidase), substrates, cofactors, inhibitors, dyes, radionuclides, luminescent groups, fluorescent groups, colloidal gold and biotin. The conjugation of binding agent to reporter group may be achieved using standard methods known to those of ordinary skill in the art. Common binding agents may also be purchased conjugated to a variety of reporter groups from many sources (*e.g.*, Zymed Laboratories, San Francisco, Calif. and Pierce, Rockford, III.).

The detection reagent is then incubated with the immobilized antibody polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti-*Leishmania* antibodies in the sample, the signal detected from the reporter group that remains bound to the solid support is generally compared to a signal that corresponds to a predetermined cut-off value. In one embodiment, the cut-off value is preferably the average mean signal obtained when the immobilized polypeptide is incubated with samples from an uninfected patient. In general, a sample generating a signal that is three standard deviations above the predetermined cut-off value is considered positive (*i.e.,* reactive with the polypeptide). In an alternate embodiment, the cut-off value is determined using a Receiver Operator Curve, according to the method of Sackett et al., Clinical Epidemiology: A Basic Science for Clinical Medicine, p. 106-7 (Little Brown and Co., 1985). Briefly, in this embodiment, the cut-off value may be determined from a plot of pairs of true positive rates (*i.e.,* sensitivity) and false positive rates (100%-specificity) that correspond to each possible cut-off value for the diagnostic test result. The cut-off value on the plot that is the closest to the upper lefthand comer (*i.e.,* the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate.

In other embodiments, an assay is performed in a flow-through assay format, wherein the antigen is immobilized on a membrane such as nitrocellulose. In the flow-through test, antibodies within the sample bind to the immobilized polypeptide as the sample passes through the membrane. A detection reagent (*e.g*., protein A-colloidal gold) then binds to the antibody-polypeptide complex as the solution containing the detection reagent flows through the membrane. The detection of bound detection reagent may then be performed as described above.

In other embodiments, an assay if performed in a strip test format, also known as a lateral flow format. Here, one end of the membrane to which polypeptide is bound is immersed in a solution containing the sample. The sample migrates along the membrane through a region containing detection reagent and to the area of immobilized fusion polypeptide. Concentration of detection reagent at the fusion polypeptide indicates the presence of *Leishmania* antibodies in the sample. Typically, the concentration of detection reagent at that site generates a pattern, such as a line, that can be read visually. The absence of such a pattern indicates a negative result. In general, the amount of fusion polypeptide immobilized on the membrane is selected to generate a visually discernible pattern when the biological sample contains a level of antibodies that would be sufficient to generate a positive signal in an ELISA, as discussed above. Preferably, the amount of fusion polypeptide immobilized on the membrane ranges from about 25 ng to about 1 µg, and more preferably from about 50 ng to about 500 ng. Such tests can typically be performed with a very small amount (*e.g*., one drop) of patient serum or blood. Lateral flow tests can operate as either competitive or sandwich assays.

In still other embodiments, a fusion polypeptide of the invention is adapted for use in a dual path platform (DPP) assay. Such assays are described, for example, in U.S. Patent No. 7,189,522.

Of course, numerous other assay protocols exist that are suitable for use with the fusion polypeptides of the present invention. It will be understood that the above descriptions are intended to be exemplary only.

The assays discussed above may be used, in certain aspects of the invention, to specifically detect visceral leishmaniasis. In this aspect, antibodies in the sample may be detected using a fusion polypeptide of the present invention, *e.g*., comprising an amino acid sequence of antigenic/immunogenic fragments or epitopes of *Leishmania* SMT and NH antigens. Preferably, the *Leishmania* antigens are immobilized by adsorption to a solid support such as a well of a microtiter plate or a membrane, as described above, in roughly similar amounts such that the total amount of fusion polypeptide in contact with the support ranges from about 10 ng to about 100 µg. The remainder of the steps in the assay may generally be performed as described above. It will be readily apparent to those of ordinary skill in the art that, by combining polypeptides described herein with other polypeptides that can detect cutaneous and mucosal leishmaniasis, the polypeptides disclosed herein may be used in methods that detect all types of leishmaniasis.

In another aspect of this invention, immobilized fusion polypeptides may be used to purify antibodies that bind thereto. Such antibodies may be prepared by any of a variety of techniques known to those of ordinary skill in the art. *See, e.g.,* Harlow and Land, Antibodies. A Laboratory Manual, Cold Spring Harbor Laboratory Press, 1988. In one such technique, an immunogen comprising a fusion polypeptide of the present invention is initially injected into any of a wide variety of mammals (*e.g*., mice, rats, rabbits, sheep and goats). In this step, the polypeptide may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic fusion polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred.

Monoclonal antibodies may be isolated from the supernatants of growing hybridoma colonies. In this process, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. One or more polypeptides may be used in the purification process in, for example, an affinity chromatography step.

Monospecific antibodies that bind to a fusion polypeptide comprising two or more immunogenic portions of *Leishmania* antigens may be used, for example, to detect *Leishmania* infection in a biological sample using one of a variety of immunoassays, which may be direct or competitive. Briefly, in one direct assay format, a monospecific antibody may be immobilized on a solid support (as described above) and contacted with the sample to be tested. After removal of the unbound sample, a second monospecific antibody, which has been labeled with a reporter group, may be added and used to detect bound antigen. In an exemplary competitive assay, the sample may be combined with the monoclonal or polyclonal antibody, which has been labeled with a suitable reporter group. The mixture of sample and antibody may then be combined with polypeptide antigen immobilized on a suitable solid support. Antibody that has not bound to an antigen in the sample is allowed to bind to the immobilized antigen and the remainder of the sample and antibody is removed. The level of antibody bound to the solid support is inversely related to the level of antigen in the sample. Thus, a lower level of antibody bound to the solid support indicates the presence of *Leishmania* in the sample. Other formats for using monospecific antibodies to detect *Leishmania* in a sample will be apparent to those of ordinary skill in the art, and the above formats are provided solely for exemplary purposes.

### EXAMPLES

### Example 1

### Fusion Polypeptide Immunogenicity and Protection against Leishmaniasis

*NS Fusion Polypeptide.* The fusion polypeptide referred to as NS (also known as LEISH F3) was generated by the tandem linkage of two *Leishmania* open reading frames encoding the proteins, nonspecific nucleoside hydrolase (NH) and sterol 24-c-methyltransferase (SMT). LEISH F3 has an amino acid sequence set forth in SEQ ID NO: 13, which contains residues 1 to 314 of the full length Leishmania infantum/donovani NH protein, and residues 2 to 353 of the full length Leishmania infantum SMT protein. The 666 amino acid fusion polypeptide has a predicted mass of 73,992 Da and was expressed in *E. coli* and purified by chromatography.
*Humoral Response.* Experiments were conducted to compare antibody levels induced against NS in the absence or presence of adjuvants in Balb/c mice. Mice were immunized intramuscularly three times 3 weeks apart with saline, NS antigen alone (10 µg), NS (10 µg) + GLA-SE (5 µg: glucopyranosyl lipid A in a stable emulsion) or NS (10 µg) + MPL-SE (20 µg; monophosphoryl lipid A in a stable emulsion). Serum was collected from immunized mice 3 weeks following the third immunization. Mice injected with NS in the absence of an adjuvant mount an NS specific antibody response that is predominantly IgG1, indicating a Th-2 like response. However, BALB/c mice injected with NS formulated in TLR4-based stable emulsion adjuvants (GLA-SE or MPL-SE) generated a strong NS-specific IgG that was predominantly IgG2a, indicating a Th1-type response (Fig.2). No antibody responses against NS were detected in saline immunized animals.
*Cellular Response.* To determine if immunization with NS induced a T cell response, antigen-specific recall responses were evaluated 4 weeks after the last boost. Splenocytes isolated from mice immunized with NS formulated in either GLA-SE or MPL-SE secreted high amounts of the Th1-type cytokine, IFN-γ in response to antigen. In contrast antigen alone group showed much weaker IFN-γ responses (Fig. 3), in concordance with the humoral response seen in these mice (Fig.2).
*Prophylactic Studies.* The NS fusion polypeptide was also evaluated with respect to protection against visceral leishmaniasis (VL) using the Balb/c mouse model. Mice were immunized subcutaneously 3 times 3 weeks apart with NS (7.4 µg or 0.74 µg) formulated with GLA-SE (5 µg) or MPL-SE (20 µg). Saline immunized animals acted as the negative control for the experiment. One month after the last immunization mice were challenged via intra-cardiac route with 5x10⁶ *L. donovani* promastigotes. Livers were harvested one month post-challenge and parasite burdens determined by limiting dilution assay. 7.4 µg of NS formulated with GLA-SE (5 µg) or MPL-SE (20 µg) resulted in 73% and 85% reductions in liver parasite burden, respectively. 0.74 µg of NS formulated as above resulted in 70% and 87% reductions in liver parasite burdens, respectively compared to the saline control group (Fig. 4).

### Example 2

### Non-Human Primate Immunogenicity and Safety Study

A multiple dose safety and efficacy study was conducted in Rhesus monkeys to evaluate the safety and immunogenicity of a Leishmania vaccine consisting of NS antigen with GLA-SE or MPL-SE, compared to antigen alone, following intramuscular administration on day 1, 29, and 57 in male and female rhesus monkeys.

**Table I: Design of non human primate study**

| **Group** | **Vaccine Route** | **Test Article** | **Total Injection Vol (µL/animal)** | **Number of Animals Female/Male¹** |
|---|---|---|---|---|
| 1 | Intramuscular | NS (20µg) | 500 | 3/3 |
| 2 | | NS (20 µg) + GLA-SE (5µg) | 500 | 3/3 |
| 3 | | NS (20 µg) + GLA-SE (10µg) | 500 | 2/4 |
| 4 | | NS (20 µg) + MPL-SE (20 µg) | 500 | 4/2 |

| | | | | |
|---|---|---|---|---|
| ¹Due to a dosing error, the number of males and females in Groups 3 and 4 was not the same NS = leishmania antigen, GLA-SE = glucopyranosyl lipid A adjuvant in SE, MPL-SE = monophosphoryl lipid A adjuvant in SE, SE = stable emulsion | | | | |

Fifteen male and fourteen female naïve Rhesus monkeys (*Macaca mulatta*) of Chinese origin (2 to 9 years old and 3 to 9 kg at pre-study examination) were randomized into four dose groups (Table I). Clinical observations were made twice daily, body weights were measured once weekly, and rectal temperatures were taken daily for three days following each dose administration. Injection sites were monitored for three days following each dose administration using a dermal observation scoring system. Clinical pathology specimens (hematology, coagulation, and serum chemistry including electrophoresis) were collected at scheduled time points. Blood samples were collected for stimulation (whole blood assay) and serum antibody assays at scheduled time points.

*Humoral responses to NS vaccine in non-human primates.* NS specific total IgG levels were evaluated in sera of individual animals for all groups of vaccinated rhesus monkeys at baseline (day -8; d-8) and subsequently 2 weeks post prime (day15; d15), 1 week post 1^{st} boost (day36; d36) and finally 1 week post 2^{nd} boost (day64; d64) by ELISA (Fig.5). None of the animals had any antibody response against NS at baseline (Day -8). In animals immunized with antigen alone (group#1) no antibody responses were detected at d15 or d36 and antibody responses were very weak on d64. In contrast, groups 2-4 containing NS in presence of an adjuvant mounted antibody responses following the first vaccination (d15) and strong antibody titers were observed in all animals at d36 (1 week post 1^{st} boost) and remained high at d64 (1 week post 2^{nd} boost).

*T cell responses to NS vaccine in non-human primates.* A whole blood assay was used to measure NS antigen specific recall responses to the vaccine in all immunized animals. Blood was collected from all animals at baseline (d-8) and two time points following vaccination; 3 weeks post 1^{st} vaccine boost (d50) and 3 weeks post 2^{nd} vaccine boost (d78). The whole blood assay was set up within two hours of blood collection. Blood was diluted 1:1 with complete RPMI and stimulated with 10ug/ml of NS antigen for 48 hr. Supernatants were clarified by centrifugation and the levels of various cytokines were measured simultaneously using the Milliplex MAP immunoassay panel for non-human primates. IFN-γ responses were extremely low or non existent at baseline (d-8) in all groups of animals (Fig.6). Weak IFN-γ responses were detected at both days 50 and 78 in animals of group 1 immunized with NS antigen alone. Strong IFN-γ responses were observed at day 50 in animals of groups #2 & 3. Animals of group #4 that had been immunized with NS and MPL-SE had comparatively weaker IFN-γ responses compared animals that had been immunized with NS and GLA-SE (groups #2, #3). Animals that had received NS with 10ug GLA-SE (group#3) showed a higher IL-5 response compared to other groups. These studies clearly demonstrate that NS induces a strong Th1-like response when formulated in TLR4-agonist based adjuvant(s), such as GLA-SE or MPL-SE.

As would be recognized by the skilled artisan, these and other changes can be made to the embodiments of the invention in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled.

## Claims

1. A fusion polypeptide comprising a *Leishmania* sterol 24-c-methyltransferase (SMT) polypeptide sequence and a *Leishmania* non-specific nucleoside hydrolase (NH) polypeptide sequence, wherein the *Leishmania* NH polypeptide sequence comprises a sequence selected from the group consisting of SEQ ID NOs: 1, 3 and 5, or a sequence having at least 90%, at least 95%, at least 96% or at least 97% identity thereto; and wherein the *Leishmania* SMT polypeptide sequence comprises a sequence selected from the group consisting of SEQ ID NOs: 7, 9 and 11, or a sequence having at least 90%, at least 95%, at least 96% or at least 97% identity thereto.

2. The fusion polypeptide of claim 1, wherein the *Leishmania* NH polypeptide sequence comprises a sequence selected from the group consisting of SEQ ID NO: 1, 3 and 5, and the *Leishmania* SMT polypeptide sequence comprises a sequence selected from the group consisting of SEQ ID NO: 7, 9 and 11.

3. The fusion polypeptide of claim 1, wherein the fusion polypeptide comprises an amino acid sequence set forth in SEQ ID NO: 13, or a sequence having at least 90%, at least 95% or at least 98% identity thereto.

4. An isolated polynucleotide encoding a fusion polypeptide of any one of claims 1 to 3.

5. A composition comprising at least one component selected from the group consisting of a fusion polypeptide of any one of claims 1 to 3 and a polynucleotide of claim 4, in combination with at least one immunostimulant.

6. The composition according to claim 5, wherein the immunostimulant is selected from the group consisting of a CpG-containing oligonucleotide, synthetic lipid A, monophosphoryl lipid A (MPL™), 3-de-O-acylated monophosphoryl lipid A (3D-MPL™), saponins, saponin mimetics, aminoalkyl glucosaminide 4-phosphates (AGPs), Toll-like receptor (TLR) agonists such as a TLR4 agonist, a TLR7/8 agonist or a TLR9 agonist, glucopyranosyl lipid adjuvant (GLA), imiquimod, gardiquimod and resiquimod or a combination thereof.

7. A composition according to claim 5 or 6 for use in method for stimulating an immune response against *Leishmania* in a mammal, wherein the fusion polypeptide or polynucleotide in the composition are incorporated into a nucleic acid expression system, or bacterial or viral expression system.

8. A method for detecting *Leishmania* infection in a biological sample, comprising:
a. contacting a biological sample with a fusion polypeptide of any one of claims 1 to 3, wherein the fusion polypeptide is optionally bound to a solid support; and
b. detecting in the biological sample the presence of antibodies that bind to the fusion polypeptide, thereby detecting *Leishmania* infection in a biological sample.

9. The method of claim 8, wherein the biological sample is selected from the group consisting of sera, blood and saliva.

10. A diagnostic reagent comprising a fusion polypeptide of any one of claims 1 to 3 immobilized on a solid support.

11. A diagnostic kit for detecting *Leishmania* infection in a biological sample comprising a fusion polypeptide of any one of claims 1 to 3, and a detection reagent.

12. The kit of claim 11, wherein the kit comprises an assay format selected from the group consisting of a lateral flow test strip assay, a dual path platform assay and an ELISA assay.

13. A point of care diagnostic kit for detecting *Leishmania* infection in a biological sample comprising a fusion polypeptide of any one of claims 1 to 3 immobilized on a solid support in a lateral flow test strip format.

## Patentansprüche

1. Fusionspolypeptid, das eine Leishmania-Sterol-24-c-methyltransferase- (-SMT-) Polypeptidsequenz und eine nichtspezifische Leishmania-Nucleosidhydrolase- (-NH-) Polypeptidsequenz umfasst, wobei die Leishmania-NH-Polypeptidsequenz eine Sequenz, die aus der aus SEQ ID NO: 1, 3 und 5 bestehenden Gruppe ausgewählt ist, oder eine Sequenz mit zumindest 90 %, zumindest 95 %, zumindest 96 % oder zumindest 97 % Identität damit umfasst; und wobei die Leishmania-SMT-Polypeptidsequenz eine Sequenz, die aus der aus SEQ ID NO: 7, 9 und 11 bestehenden Gruppe ausgewählt ist, oder eine Sequenz mit zumindest 90 %, zumindest 95 %, zumindest 96 % oder zumindest 97 % Identität damit umfasst.

2. Fusionspolypeptid nach Anspruch 1, wobei die Leishmania-NH-Polypeptidsequenz eine Sequenz umfasst, die aus der aus SEQ ID NO: 1, 3 und 5 bestehenden Gruppe ausgewählt ist, und die Leishmania-SMT-Polypeptidsequenz eine Sequenz umfasst, die aus der aus SEQ ID NO: 7, 9 und 11 bestehenden Gruppe ausgewählt ist.

3. Fusionspolypeptid nach Anspruch 1, wobei das Fusionspolypeptid eine in SEQ ID NO: 13 dargelegte Aminosäuresequenz oder eine Sequenz mit zumindest 90 %, zumindest 95 % oder zumindest 98 % Identität damit umfasst.

4. Isoliertes Polynucleotid, das für ein Fusionspolypeptid nach einem der Ansprüche 1 bis 3 kodiert.

5. Zusammensetzung, die zumindest eine Komponente, die aus der aus einem Fusionspolypeptid nach einem der Ansprüche 1 bis 3 und einem Polynucleotid nach Anspruch 4 bestehenden Gruppe ausgewählt ist, in Kombination mit zumindest einem Immunstimulans umfasst.

6. Zusammensetzung nach Anspruch 5, wobei das Immunstimulans aus der aus einem CpG-hältigen Oligonucleotid, einem synthetischen Lipid A, Monophosphoryllipid A (MPL™), 3-de-O-acetyliertem Monophosphoryllipid A (3D-MPL™), Saponinen, Saponinmimetika, Aminoalkylglucosaminid-4-phosphaten (AGPs), Agonisten von Toll-like Rezeptoren (TLR) wie einem TLR4-Agonisten, einem TLR7/8-Agonisten oder einem TLR9-Agonisten, einem Glucopyranosyllipidadjuvans (GLA), Imiquimod, Gardiquimod und Resiquimod oder einer Kombination davon bestehenden Gruppe ausgewählt ist.

7. Zusammensetzung nach Anspruch 5 oder 6 zur Verwendung in einem Verfahren zur Stimulation einer Immunantwort gegen Leishmania in einem Säugetier, wobei das Fusionspolypeptid oder Polynucleotid in der Zusammensetzung in ein Nucleinsäureexpressionssystem oder ein bakterielles oder virales Expressionssystem inkorporiert sind.

8. Verfahren zum Nachweis einer Leishmania-Infektion in einer biologischen Probe, das Folgendes umfasst:
a. Kontaktieren einer biologischen Probe mit einem Fusionspolypeptid nach einem der Ansprüche 1 bis 3, wobei das Fusionspolypeptid gegebenenfalls an einen festen Träger gebunden ist; und
b. Nachweisen der Gegenwart von Antikörpern, die an das Fusionspolypeptid binden, in der biologischen Probe, um so eine Leishmania-Infektion in einer biologischen Probe nachzuweisen.

9. Verfahren nach Anspruch 8, wobei die biologische Probe aus der aus Serum, Blut und Speichel bestehenden Gruppe ausgewählt ist.

10. Diagnosereagens, das ein Fusionspolypeptid nach einem der Ansprüche 1 bis 3 auf einem festen Träger immobilisiert umfasst.

11. Diagnoseset zum Nachweis einer Leishmania-Infektion in einer biologischen Probe, das ein Fusionspolypeptid nach einem der Ansprüche 1 bis 3 und ein Nachweisreagens umfasst.

12. Set nach Anspruch 11, wobei das Set ein Assayformat umfasst, das aus der aus einem Lateralfluss-Teststreifenassay, einem Zweiwege-Plattform-Assay und einem ELISA-Assay bestehenden Gruppe ausgewählt ist.

13. Patientennahes Sofortdiagnoseset zum Nachweis einer Leishmania-Infektion in einer biologischen Probe, das ein Fusionspolypeptid nach einem der Ansprüche 1 bis 3 auf einem festen Träger immobilisiert im Lateralfluss-Teststreifenformat umfasst.

## Revendications

1. Polypeptide de fusion comprenant une séquence polypeptidique de stérol 24-c-méthyltransférase (SMT) de *Leishmania* et une séquence polypeptidique de nucléoside hydrolase (NH) non spécifique de *Leishmania,* dans lequel la séquence polypeptidique de NH de *Leishmania* comprend une séquence choisie dans le groupe comprenant SEQ ID NO : 1, 3 et 5, ou une séquence ayant au moins 90 %, au moins 95 %, au moins 96 % ou au moins 97 % d'identité avec celle-ci ; et
dans lequel la séquence polypeptidique de SMT de *Leishmania* comprend une séquence choisie dans le groupe comprenant SEQ ID NO : 7, 9 et 11, ou une séquence ayant au moins 90 %, au moins 95 %, au moins 96 % ou au moins 97 % d'identité avec celle-ci.

2. Polypeptide de fusion selon la revendication 1, dans lequel la séquence polypeptidique de NH de *Leishmania* comprend une séquence choisie dans le groupe comprenant SEQ ID NO : 1, 3 et 5, et la séquence polypeptidique de SMT de *Leishmania* comprend une séquence choisie dans le groupe comprenant SEQ ID NO : 7, 9 et 11.

3. Polypeptide de fusion selon la revendication 1, dans lequel le polypeptide de fusion comprend une séquence d'acides aminés établie dans SEQ ID NO : 13, ou une séquence ayant au moins 90 %, au moins 95 % ou au moins 98 % d'identité avec celle-ci.

4. Polynucléotide isolé codant pour un polypeptide de fusion selon l'une quelconque des revendications 1 à 3.

5. Composition comprenant au moins un composant choisi dans le groupe comprenant un polypeptide de fusion selon l'une quelconque des revendications 1 à 3 et un polynucléotide selon la revendication 4, en combinaison avec au moins un immunostimulant.

6. Composition selon la revendication 5, dans laquelle l'immunostimulant est choisi dans le groupe comprenant un oligonucléotide contenant CpG, un lipide A synthétique, un lipide A de monophosphoryle (MPL™), un lipide A de monophosphoryle 3-dés-O-acylé (3D-MPL™), des saponines, des mimétiques de saponine, des aminoalkyle glucosaminide 4-phosphates (AGPs), des agonistes de récepteur de type Toll (TLR) tels qu'un agoniste TLR4, un agoniste TLR7/8 ou un agoniste TLR9, un adjuvant lipidique à base de glucopyranosyle (GLA), de l'imiquimod, du gardimiquimod et du résiquimod ou une combinaison de ceux-ci.

7. Composition selon la revendication 5 ou 6 pour une utilisation dans un procédé de stimulation d'une réponse immunitaire à l'encontre de la *leishmania* chez un mammifère, dans laquelle le polypeptide de fusion ou le polynucléotide dans la composition sont incorporés dans un système d'expression d'acide nucléique, ou un système d'expression bactérien ou viral.

8. Procédé de détection d'une infection par *Leishmania* dans un échantillon biologique, comprenant :
a. la mise en contact d'un échantillon biologique avec un polypeptide de fusion selon l'une quelconque des revendications 1 à 3, dans lequel le polypeptide de fusion est facultativement lié à un support solide ; et
b. la détection dans l'échantillon biologique de la présence d'anticorps qui se lient au polypeptide de fusion, en détectant ainsi une infection par *Leishmania* dans un échantillon biologique.

9. Procédé selon la revendication 8, dans lequel l'échantillon biologique est choisi dans le groupe constitué de sérums, de sang et de salive.

10. Réactif de diagnostic comprenant un polypeptide de fusion selon l'une quelconque des revendications 1 à 3 immobilisé sur un support solide.

11. Kit de diagnostic pour détecter une infection par *Leishmania* dans un échantillon biologique comprenant un polypeptide de fusion selon l'une quelconque des revendications 1 à 3, et un réactif de détection.

12. Kit selon la revendication 11, dans lequel le kit comprend un format de d'essai choisi dans le groupe consistant en un essai sur bandelette de test en flux latéral, un essai sur plate-forme à double voie et un essai ELISA.

13. Kit de diagnostic d'emplacement de soin pour détecter une infection par *Leishmania* dans un échantillon biologique comprenant un polypeptide de fusion selon l'une quelconque des revendications 1 à 3 immobilisé sur un support solide dans un format de bandelette de test à flux latéral.


### SEQUENCE LISTING

<110> Infectious Disease Research Institute
   Bhatia, Ajay
   Reed, Steven G.
<120> VACCINES COMPRISING NON-SPECIFIC
   NUCLEOSIDE HYDROLASE AND STEROL 24-C-METHYLTRANSFERASE (SMT) POLYPEPTIDES FOR THE TREATMENT AND DIAGNOSIS OF LEISHMANIASIS
<130> 480239.425PC
<140> PCT/US
   <141> 2011-11-07
<150> 61/411,366
   <151> 2010-11-08
<160> 14
<170> FastSEQ for Windows Version 4.0
<210> 1
   <211> 314
   <212> PRT
   <213> Leishmania infantum
<400> 1
   Met Pro Arg Lys Ile Ile Leu Asp Cys Asp Pro Gly Ile Asp Asp Ala
    1               5                  10                  15
   Val Ala Ile Phe Leu Ala His Gly Asn Pro Glu Val Glu Leu Leu Ala
               20                  25                  30
   Ile Thr Thr Val Val Gly Asn Gln Thr Leu Glu Lys Val Thr Arg Asn
           35                  40                  45
   Ala Arg Leu Val Ala Asp Val Ala Gly Ile Val Gly Val Pro Val Ala
       50                  55                  60
   Ala Gly Cys Thr Lys Pro Leu Val Arg Gly Val Arg Asn Ala Ser Gln
   65                  70                  75                  80
   Ile His Gly Glu Thr Gly Met Gly Asn Val Ser Tyr Pro Pro Glu Phe
                   85                  90                  95
   Lys Thr Lys Leu Asp Gly Arg His Ala Val Gln Leu Ile Ile Asp Leu
               100                 105                 110
   Ile Met Ser His Glu Pro Lys Thr Ile Thr Leu Val Pro Thr Gly Gly
           115                 120                 125
   Leu Thr Asn Ile Ala Met Ala Val Arg Leu Glu Pro Arg Ile Val Asp
       130                 135                 140
   Arg Val Lys Glu Val Val Leu Met Gly Gly Gly Tyr His Thr Gly Asn
   145                 150                 155                 160
   Ala Ser Pro Val Ala Glu Phe Asn Val Phe Val Asp Pro Glu Ala Ala
                   165                 170                 175
   His Ile Val Phe Asn Glu Ser Trp Asn Val Thr Met Val Gly Leu Asp
               180                 185                 190
   Leu Thr His Gln Ala Leu Ala Thr Pro Ala Val Gln Lys Arg Val Lys
           195                 200                 205
   Glu Val Gly Thr Lys Pro Ala Ala Phe Met Leu Gln Ile Leu Asp Phe
       210                 215                 220
   Tyr Thr Lys Val Tyr Glu Lys Glu Arg Asn Thr Tyr Ala Thr Val His
   225                 230                 235                 240
   Asp Pro Cys Ala Val Ala Tyr Val Ile Asp Pro Thr Val Met Thr Thr
                   245                 250                 255
   Glu Gln Val Pro Val Asp Ile Glu Leu Asn Gly Ala Leu Thr Thr Gly
               260                 265                 270
   Met Thr Val Ala Asp Phe Arg Tyr Pro Arg Pro Lys His Cys His Thr
           275                 280                 285
   Gln Val Ala Val Lys Leu Asp Phe Asp Lys Phe Trp Cys Leu Val Ile
       290                 295                 300
   Asp Ala Leu Lys Arg Ile Gly Asp Pro Gln
   305                 310
<210> 2
   <211> 942
   <212> DNA
   <213> Leishmania infantum
<400> 2
   atgccgcgca agattattct cgattgtgat cccgggatcg atgatgccgt ggccatcttt 60
   ctcgcccacg gcaacccgga ggtcgagctg ctggccatta cgacggtggt gggcaaccag 120
   accctggaga aggtgacccg gaacgcgcgg ctggtagctg acgtagccgg catcgttggt 180
   gtgcccgtcg cggctggttg caccaagccc ctcgtgcgcg gtgtgcggaa tgcctctcag 240
   attcatggcg aaaccggcat gggtaacgtc tcctacccac cagagttcaa gacaaagttg 300
   gacggccgcc atgcagtgca gctgatcatc gaccttatca tgtcgcacga gccgaagacg 360
   atcacgcttg tgcctacggg tggcctgacg aacattgcga tggctgtccg tcttgagccg 420
   cgcatcgtgg accgtgtgaa ggaggtggtt ctgatgggtg gcggctacca tactggtaat 480
   gcgtcccctg tagcggagtt caacgtcttc gtcgacccgg aggcggcgca cattgtgttc 540
   aacgagagct ggaacgtaac gatggtgggg ctggacctaa cgcaccaggc actcgccacg 600
   ccggcggtcc agaagcgagt gaaggaggtg ggcacgaagc cggctgcctt catgctgcag 660
   attttggact tttacacgaa ggtgtacgaa aaggagcgca acacgtacgc gacggtgcac 720
   gatccctgcg ctgtggcgta cgtgattgac cccaccgtga tgacgacgga gcaagtgcca 780
   gtggacatcg aactcaatgg ggcactgacg actgggatga cggtcgcgga cttccgctac 840
   ccacggccaa agcactgcca cacgcaggtg gctgtgaagc tggacttcga caagttttgg 900
   tgcctcgtga ttgacgcact caagcgcatc ggcgatcctc aa                   942
<210> 3
   <211> 314
   <212> PRT
   <213> Leishmania donovani
<400> 3
   Met Pro Arg Lys Ile Ile Leu Asp Cys Asp Pro Gly Ile Asp Asp Ala
    1               5                   10                  15
   Val Ala Ile Phe Leu Ala His Gly Asn Pro Glu Val Glu Leu Leu Ala
               20                  25                  30
   Ile Thr Thr Val Val Gly Asn Gln Thr Leu Glu Lys Val Thr Arg Asn
           35                  40                  45
   Ala Arg Leu Val Ala Asp Val Ala Gly Ile Val Gly Val Pro Val Ala
       50                  55                  60
   Ala Gly Cys Thr Lys Pro Leu Val Arg Gly Val Arg Asn Ala Ser Gln
   65                  70                  75                  80
   Ile His Gly Glu Thr Gly Met Gly Asn Val Ser Tyr Pro Pro Glu Phe
                   85                  90                  95
   Lys Thr Lys Leu Asp Gly Arg His Ala Val Gln Leu Ile Ile Asp Leu
               100                 105                 110
   Ile Met Ser His Glu Pro Lys Thr Ile Thr Leu Val Pro Thr Gly Gly
           115                 120                 125
   Leu Thr Asn Ile Ala Met Ala Val Arg Leu Glu Pro Arg Ile Val Asp
       130                 135                 140
   Arg Val Lys Glu Val Val Leu Met Gly Gly Gly Tyr His Thr Gly Asn
   145                 150                 155                 160
   Ala Ser Pro Val Ala Glu Phe Asn Val Phe Val Asp Pro Glu Ala Ala
                   165                 170                 175
   His Ile Val Phe Asn Glu Ser Trp Asn Val Thr Met Val Gly Leu Asp
               180                 185                 190
   Leu Thr His Gln Ala Leu Ala Thr Pro Ala Val Gln Lys Arg Val Lys
               195                 200                 205
   Glu Val Gly Thr Lys Pro Ala Ala Phe Met Leu Gln Ile Leu Asp Phe
           210                 215                 220
   Tyr Thr Lys Val Tyr Glu Lys Glu Arg Asn Thr Tyr Ala Thr Val His
   225                 230                 235                 240
   Asp Pro Cys Ala Val Ala Tyr Val Ile Asp Pro Thr Val Met Thr Thr
                   245                 250                 255
   Glu Gln Val Pro Val Asp Ile Glu Leu Asn Gly Ala Leu Thr Thr Gly
               260                 265                 270
   Met Thr Val Ala Asp Phe Arg Tyr Pro Arg Pro Lys His Cys His Thr
           275                 280                 285
   Gln Val Ala Val Lys Leu Asp Phe Asp Lys Phe Trp Cys Leu Val Ile
       290                 295                 300
   Asp Ala Leu Lys Arg Ile Gly Asp Pro Gln
   305                 310
<210> 4
   <211> 942
   <212> DNA
   <213> Leishmania donovani
<400> 4
   atgccgcgca agattattct cgattgtgat cccgggatcg atgatgccgt ggccatcttt 60
   ctcgcccacg gcaacccgga ggtcgagctg ctggccatta cgacggtggt gggcaaccag 120
   accctggaga aggtgacccg gaacgcgcgg ctggtagctg acgtagccgg catcgttggt 180
   gtgcccgtcg cggctggttg caccaagccc ctcgtgcgcg gtgtgcggaa tgcctctcag 240
   attcatggcg aaaccggcat gggtaacgtc tcctacccac cagagttcaa gacaaagttg 300
   gacggccgtc atgcagtgca gctgatcatc gaccttatca tgtcgcacga gccgaagacg 360
   atcacgcttg tgcctacggg tggcctgacg aacattgcga tggctgtccg tcttgagccg 420
   cgcatcgtgg accgtgtgaa ggaggtggtt ctgatgggtg gcggctacca tactggtaat 480
   gcgtcccctg tagcggagtt caacgtcttc gtcgacccgg aggcggcgca cattgtgttc 540
   aacgagagct ggaacgtaac gatggtgggg ctggacctaa cgcaccaggc actcgccacg 600
   ccggcggtcc agaagcgagt gaaggaggtg ggcacgaagc cggctgcctt catgctgcag 660
   attttggact tttacacgaa ggtgtacgaa aaggagcgca acacgtacgc gacggtgcac 720
   gatccctgcg ctgtggcgta cgtgattgac cccaccgtga tgacgacgga gcaagtgcca 780
   gtggacatcg agctcaatgg ggcactgacg actgggatga cggtcgcgga cttccgctac 840
   ccacggccaa agcactgcca cacgcaggtg gctgtgaagc tggacttcga caagttttgg 900
   tgcctcgtga ttgacgcact caagcgcatc ggcgatcctc aa                   942
<210> 5
   <211> 314
   <212> PRT
   <213> Leishmania major
<400> 5
   Met Pro Arg Lys Ile Ile Leu Asp Cys Asp Pro Gly Ile Asp Asp Ala
    1               5                  10                  15
   Val Ala Ile Phe Leu Ala His Gly Asn Pro Glu Ile Glu Leu Leu Ala
               20                  25                  30
   Ile Thr Thr Val Val Gly Asn Gln Ser Leu Glu Lys Val Thr Gln Asn
           35                  40                  45
   Ala Arg Leu Val Ala Asp Val Ala Gly Ile Val Gly Val Pro Val Ala
       50                  55                  60
   Ala Gly Cys Thr Lys Pro Leu Val Arg Gly Val Arg Asn Ala Ser His
   65                  70                  75                  80
   Ile His Gly Glu Thr Gly Met Gly Asn Val Ser Tyr Pro Pro Glu Phe
                   85                  90                  95
   Lys Thr Lys Leu Asp Gly Arg His Ala Val Gln Leu Ile Ile Asp Leu
               100                 105                 110
   Ile Met Ser His Glu Pro Lys Thr Ile Thr Leu Val Pro Thr Gly Gly
           115                 120                 125
   Leu Thr Asn Ile Ala Met Ala Val Arg Leu Glu Pro Arg Ile Val Asp
       130                 135                 140
   Arg Val Lys Glu Val Val Leu Met Gly Gly Gly Tyr His Thr Gly Asn
   145                 150                 155                 160
   Ala Ser Pro Val Ala Glu Phe Asn Val Phe Ile Asp Pro Glu Ala Ala
                   165                 170                 175
   His Ile Val Phe Asn Glu Ser Trp Asn Val Thr Met Val Gly Leu Asp
               180                 185                 190
   Leu Thr His Gln Ala Leu Ala Thr Pro Ala Val Gln Lys Arg Val Arg
           195                 200                 205
   Glu Val Gly Thr Lys Pro Ala Ala Phe Met Leu Gln Ile Leu Asp Phe
       210                 215                 220
   Tyr Thr Lys Val Tyr Glu Lys Glu His Asp Thr Tyr Gly Lys Val His
   225                 230                 235                 240
   Asp Pro Cys Ala Val Ala Tyr Val Ile Asp Pro Thr Val Met Thr Thr
                   245                 250                 255
   Glu Arg Val Pro Val Asp Ile Glu Leu Asn Gly Ala Leu Thr Thr Gly
               260                 265                 270
   Met Thr Val Ala Asp Phe Arg Tyr Pro Arg Pro Lys Asn Cys Arg Thr
           275                 280                 285
   Gln Val Ala Val Lys Leu Asp Phe Asp Lys Phe Trp Cys Leu Val Ile
       290                 295                 300
   Asp Ala Leu Glu Arg Ile Gly Asp Pro Gln
   305                 310
<210> 6
   <211> 942
   <212> DNA
   <213> Leishmania major
<400> 6
   atgccgcgca agattattct cgactgtgat cccgggatcg atgatgccgt ggccatcttt 60
   ctcgcccacg gcaacccgga gatcgagctg ctggccatta cgacggtggt gggcaaccag 120
   agcctggaga aggtgaccca gaacgcgcgg ctggtagctg acgtagctgg catcgttggt 180
   gtgcccgtcg cggctggctg caccaagccc ctcgtacgcg gtgtgcggaa tgcctctcat 240
   attcacggcg aaaccggcat gggtaatgtc tcctacccac cagagttcaa gacaaagttg 300
   gacggccgcc atgcagtgca gctgatcatc gaccttatca tgtcgcacga gcccaagacg 360
   atcacgcttg tgcctacggg tggactgacg aacattgcga tggctgtccg tcttgagccg 420
   cgcatcgtgg accgtgtgaa ggaggtggtt ctgatgggtg gcggctacca tactggtaat 480
   gcttcccctg tcgcggagtt caacgtcttc atcgacccgg aggcggcgca cattgtgttc 540
   aacgagagct ggaacgtaac aatggtggga ctggacctga cgcaccaggc cctcgccacg 600
   ccggcggtcc agaagcgggt gagggaggtg ggcacgaagc cggctgcctt catgctgcag 660
   attttggact tttacacgaa ggtgtacgaa aaggagcacg acacgtacgg gaaggtgcac 720
   gatccgtgcg ctgtggcgta cgtgattgac cccaccgtga tgacgacgga gcgagtgcct 780
   gtggacatcg agctcaatgg ggcactgacg actgggatga cggtcgcgga cttccgctac 840
   ccgcggccaa agaactgccg cacgcaggtg gctgtgaagc tggacttcga caagttttgg 900
   tgcctcgtga ttgacgcact cgagcgcatc ggcgatcctc ag                   942
<210> 7
   <211> 353
   <212> PRT
   <213> Leishmania infantum
<400> 7
   Met Ser Ala Gly Gly Arg Glu Thr Ala Pro Thr Asn Leu Ile Arg Arg
   1               5                   10                  15
   Arg Asn Lys Asp Glu Thr Asn Gly Asp Val Ser Ala Ala Ala Asp Arg
               20                  25                  30
   Phe Arg Asp Arg Phe Glu Lys Ala Thr Leu Glu Glu Arg Lys Ala Ala
           35                  40                  45
   Thr Thr Thr Met Val Asn Glu Tyr Tyr Asp Leu Val Thr Asp Phe Tyr
       50                  55                  60
   Glu Tyr Gly Trp Gly Gln Asn Phe His Phe Ala Pro Arg Tyr Ala Gly
   65                  70                  75                  80
   Glu Thr Phe Phe Glu Ser Leu Ala Arg His Glu Tyr Phe Leu Ala Ala
                   85                  90                  95
   Arg Gly Gly Phe Met Glu Gly Asp His Ile Val Asp Val Gly Cys Gly
               100                 105                 110
   Val Gly Gly Pro Ala Arg Asn Met Val Arg Leu Thr Arg Cys Asn Val
           115                 120                 125
   Ile Gly Val Asn Asn Asn Asp Tyr Gln Ile Ser Arg Ala Arg Arg His
       130                 135                 140
   Asp Ala Leu Ala Gly Met Ser Ser Lys Ile Asp Tyr Val Lys Thr Asp
   145                 150                 155                 160
   Phe Cys Asn Met Ser Leu Ala Asp Asn Thr Phe Asp Gly Ala Tyr Ala
                   165                 170                 175
   Ile Glu Ala Thr Cys His Ala Lys Asp Lys Val Lys Cys Tyr Ser Glu
               180                 185                 190
   Val Phe Arg Val Ile Lys Pro Gly Thr Cys Phe Val Leu Tyr Glu Trp
           195                 200                 205
   Cys Met Thr Asp Lys Tyr Asn Pro Asn Asp Glu Tyr His Arg Thr Ile
       210                 215                 220
   Lys His Arg Ile Glu Leu Gly Asp Gly Leu Pro Glu Met Glu Thr Cys
   225                 230                 235                 240
   Lys Gln Val Ile Glu Tyr Met Lys Gln Ala Gly Phe Val Val Glu Glu
                   245                 250                 255
   Ala Ile Asp Val Ile Ser Gln Phe Glu Ser Ser Pro Ile Lys Ser Ile
               260                 265                 270
   Pro Trp Tyr Gln Pro Leu Val Gly Asp Tyr Ser Ser Leu Gln Gly Leu
           275                 280                 285
   Arg Ser Thr Pro Ile Gly Arg Ile Leu Thr Asn Val Met Cys Arg Val
       290                 295                 300
   Leu Glu Phe Val Arg Leu Ala Pro Lys Gly Thr Tyr Lys Ala Thr Glu
   305                 310                 315                 320
   Ile Leu Glu Glu Ala Ala Glu Ser Leu Val Val Gly Gly Gln Leu Gly
                   325                 330                 335
   Ile Phe Thr Pro Ser Phe Tyr Ile Arg Ala Arg Lys Pro Ser Lys Gln
               340                 345                 350
   Ala
<210> 8
   <211> 1059
   <212> DNA
   <213> Leishmania infantum
<400> 8
   atgtccgccg gtggccgtga gaccgcgccg acgaacctga ttcgtcgccg caacaaggac 60
   gagacaaacg gggatgtcag cgccgccgcc gaccgcttcc gcgaccgctt cgagaaggca 120
   accctcgagg agcgcaaggc cgccaccacg acgatggtca acgagtacta cgacctggtg 180
   acggacttct acgagtacgg ctggggccag aacttccatt tcgcgccgcg ctacgccggc 240
   gagaccttct tcgagtccct cgcgcgccac gagtacttcc tggccgctcg cggcggcttc 300
   atggagggcg accacatcgt cgacgtgggc tgcggcgtcg gcggtccggc gcgcaacatg 360
   gttcgcctca cgcgctgcaa cgtcatcggc gtcaacaaca acgattacca gatcagccgc 420
   gctcgccgtc atgacgcgct cgccggtatg agctccaaga tcgactacgt caagaccgac 480
   ttctgcaaca tgagcttagc cgacaacacc ttcgacggcg cctacgccat cgaggccacc 540
   tgccacgcaa aggacaaggt caagtgctat agcgaggtct tccgtgtcat caagcccggc 600
   acctgctttg tcctgtacga gtggtgcatg accgacaagt acaaccccaa tgacgagtac 660
   caccgcacaa tcaagcaccg catcgagctg ggcgacggcc tgccggagat ggagacgtgc 720
   aaacaggtga tcgagtacat gaagcaggcc ggcttcgtgg tggaggaggc catagacgtc 780
   atcagtcagt tcgagtccag ccccatcaag agtatcccgt ggtaccagcc gctggtcggc 840
   gactattcgt ccctgcaggg cctgcgctct accccgattg gccgcatcct cacgaacgtc 900
   atgtgtcgcg tgctggagtt cgtgcgccta gctccgaagg gcacgtacaa ggcgacggag 960
   attttggagg aggctgcgga aagcctggtg gtgggcggtc agctcggcat cttcacgccg 1020
   tccttctaca tccgcgctcg caagccgtcc aagcaggct                        1059
<210> 9
   <211> 353
   <212> PRT
   <213> Leishmania donovani
<400> 9
   Met Ser Ala Gly Gly Arg Glu Thr Ala Pro Thr Asn Leu Ile Arg Arg
    1               5                  10                  15
   Arg Asn Lys Asp Glu Thr Asn Gly Asp Val Ser Ala Ala Ala Asp Arg
               20                  25                  30
   Phe Arg Asp Arg Phe Glu Lys Ala Thr Leu Glu Glu Arg Lys Ala Ala
           35                  40                  45
   Thr Thr Thr Met Val Asn Glu Tyr Tyr Asp Leu Val Thr Asp Phe Tyr
       50                  55                  60
   Glu Tyr Gly Trp Gly Gln Asn Phe His Phe Ala Pro Arg Tyr Ala Gly
   65                  70                  75                  80
   Glu Thr Phe Phe Glu Ser Leu Ala Arg His Glu Tyr Phe Leu Ala Ala
                   85                  90                  95
   Arg Gly Gly Phe Met Glu Gly Asp His Ile Val Asp Val Gly Cys Gly
               100                 105                 110
   Val Gly Gly Pro Ala Arg Asn Met Val Arg Leu Thr Arg Cys Asn Val
           115                 120                 125
   Ile Gly Val Asn Asn Asn Asp Tyr Gln Ile Ser Arg Ala Arg Arg His
       130                 135                 140
   Asp Ala Leu Ala Gly Met Ser Ser Lys Ile Asp Tyr Val Lys Thr Asp
   145                 150                 155                 160
   Phe Cys Asn Met Ser Leu Ala Asp Asn Thr Phe Asp Gly Ala Tyr Ala
                   165                 170                 175
   Ile Glu Ala Thr Cys His Ala Lys Asp Lys Val Lys Cys Tyr Ser Glu
               180                 185                 190
   Val Phe Arg Val Ile Lys Pro Gly Thr Cys Phe Val Leu Tyr Glu Trp
           195                 200                 205
   Cys Met Thr Asp Lys Tyr Asn Pro Asn Asp Glu Tyr His Arg Thr Ile
       210                 215                 220
   Lys His Arg Ile Glu Leu Gly Asp Gly Leu Pro Glu Met Glu Thr Cys
   225                 230                 235                 240
   Lys Gln Val Ile Glu Tyr Met Lys Gln Ala Gly Phe Val Val Glu Glu
                   245                 250                 255
   Ala Ile Asp Val Ile Ser Gln Phe Glu Ser Ser Pro Ile Lys Ser Ile
               260                 265                 270
   Pro Trp Tyr Gln Pro Leu Val Gly Asp Tyr Ser Ser Leu Gln Gly Leu
           275                 280                 285
   Arg Ser Thr Pro Ile Gly Arg Ile Leu Thr Asn Val Met Cys Arg Val
       290                 295                 300
   Leu Glu Phe Val Arg Leu Ala Pro Lys Gly Thr Tyr Lys Ala Thr Glu
   305                 310                 315                 320
   Val Leu Glu Glu Ala Ala Glu Ser Leu Val Val Gly Gly Gln Leu Gly
                   325                 330                 335
   Ile Phe Thr Pro Ser Phe Tyr Ile Arg Ala Arg Lys Pro Ser Lys Gln
               340                 345                 350
   Ala
<210> 10
   <211> 1059
   <212> DNA
   <213> Leishmania donovani
<400> 10
   atgtccgccg gtggccgtga gaccgcgccg acgaacctga ttcgtcgccg caacaaggac 60
   gagacaaacg gggatgtcag cgccgccgcc gaccgcttcc gcgaccgctt cgagaaggca 120
   accctcgagg agcgcaaggc cgccaccacg acgatggtca acgagtacta cgacctggtg 180
   acggacttct acgagtacgg ctggggccag aacttccatt tcgcgccgcg ctacgccggc 240
   gagaccttct tcgagtccct cgcgcgccac gagtacttcc tggccgctcg cggcggcttc 300
   atggagggcg accacatcgt cgacgtgggc tgcggcgtcg gcggtccggc gcgcaacatg 360
   gttcgcctca cgcgctgcaa cgtcatcggc gtcaacaaca acgattacca gatcagccgc 420
   gctcgccgtc atgacgcgct cgccggtatg agctccaaga tcgactacgt caagaccgac 480
   ttctgcaaca tgagcttagc cgacaacacc ttcgacggcg cctacgccat cgaggccacc 540
   tgccacgcaa aggacaaggt caagtgctat agcgaggtct tccgtgtcat caagcccggc 600
   acctgctttg tcctgtacga gtggtgcatg accgacaagt acaaccccaa tgacgagtac 660
   caccgcacaa tcaagcaccg catcgagctg ggcgacggcc tgccggagat ggagacgtgc 720
   aaacaggtga tcgagtacat gaagcaggcc ggcttcgtgg tggaggaggc catagacgtc 780
   atcagtcagt tcgaatccag ccccatcaag agtatcccgt ggtaccagcc gctggtcggc 840
   gactattcgt ccctgcaggg cctgcgctct accccgattg gccgcatcct cacgaacgtc 900
   atgtgtcgcg tgctggagtt cgtgcgccta gctccgaagg gcacgtacaa ggcgacggag 960
   gttttggagg aggctgcgga aagcctggtg gtgggcggtc agctcggcat cttcacgccg 1020
   tccttctaca tccgcgctcg caagccgtcc aagcaggct                        1059
<210> 11
   <211> 353
   <212> PRT
   <213> Leishmania major
<400> 11
   Met Ser Ala Gly Gly Arg Glu Thr Ala Pro Met Asn Leu Leu Arg Arg
    1               5                  10                  15
   Arg Asn Lys Asp Glu Ile Asn Gly Asp Val Asn Ala Ala Ala Asp Arg
               20                  25                  30
   Phe Arg Asn Arg Phe Glu Lys Ala Thr Leu Glu Glu Arg Lys Ala Ala
           35                  40                  45
   Thr Thr Thr Met Val Asn Glu Tyr Tyr Asp Leu Val Thr Asp Phe Tyr
       50                  55                  60
   Glu Tyr Gly Trp Gly Gln Asn Phe His Phe Ala Pro Arg Tyr Ala Gly
   65                  70                  75                  80
   Glu Thr Phe Phe Glu Ser Leu Ala Arg His Glu Tyr Phe Leu Ala Ala
                   85                  90                  95
   Arg Gly Gly Phe Met Glu Gly Asp His Ile Val Asp Val Gly Cys Gly
               100                 105                 110
   Val Gly Gly Pro Ala Arg Asn Ile Val Arg Leu Thr Arg Cys Asn Val
           115                 120                 125
   Thr Gly Val Asn Asn Asn Asp Tyr Gln Ile Ser Arg Ala Arg Arg His
       130                 135                 140
   Asp Ala Leu Ala Gly Met Ser Cys Lys Ile Asp Tyr Val Lys Thr Asp
   145                 150                 155                 160
   Phe Cys Asn Met Ser Leu Ala Asp Asn Thr Phe Asp Gly Ala Tyr Ala
                   165                 170                 175
   Ile Glu Ala Thr Cys His Ala Lys Asp Lys Val Lys Cys Tyr Ser Glu
               180                 185                 190
   Val Phe Arg Val Ile Lys Pro Gly Thr Cys Phe Val Leu Tyr Glu Trp
           195                 200                 205
   Cys Met Thr Asp Lys Tyr Asn Pro Asn Asp Glu Tyr His Arg Thr Ile
       210                 215                 220
   Lys His Arg Ile Glu Leu Gly Asp Gly Leu Pro Glu Met Glu Thr Cys
   225                 230                 235                 240
   Lys Gln Val Ile Glu Tyr Met Lys Glu Ala Gly Phe Val Val Glu Glu
                   245                 250                 255
   Ala Ile Asp Val Ile Ser Gln Phe Glu Ser Ser Pro Ile Lys Ser Ile
               260                 265                 270
   Pro Trp Tyr Gln Pro Leu Val Gly Asp Tyr Ser Ser Leu Gln Gly Leu
           275                 280                 285
   Arg Ser Thr Pro Ile Gly Arg Ile Leu Thr Asn Ile Met Cys Arg Val
       290                 295                 300
   Leu Glu Phe Val His Leu Ala Pro Lys Gly Thr Tyr Lys Ala Thr Glu
   305                 310                 315                 320
   Val Leu Glu Glu Ala Ala Glu Ser Leu Val Val Gly Gly Gln Leu Gly
                   325                 330                 335
   Ile Phe Thr Pro Ser Phe Tyr Ile Arg Ala Arg Lys Pro Ser Lys Gln
               340                 345                 350
   Ala
<210> 12
   <211> 1059
   <212> DNA
   <213> Leishmania major
<400> 12
   atgtctgccg gtggccgtga gaccgcgccg atgaacctgc ttcgtcgccg caacaaggat 60
   gagataaacg gggatgtcaa cgccgccgcc gaccgcttcc gcaaccgctt cgagaaggca 120
   accctcgagg agcgcaaggc cgccaccacg acgatggtca acgagtacta cgacctggtg 180
   acggacttct acgagtacgg ctggggccag aactttcatt tcgcgccgcg ctacgccggc 240
   gagaccttct tcgagtccct cgcgcgccac gagtacttcc tggccgcccg cggcggcttc 300
   atggagggcg accatatcgt cgacgtgggc tgcggcgtcg gcggtccggc gcgcaacata 360
   gttcgcctca cgcgctgtaa cgtcaccggc gtcaacaaca acgattacca aatcagccgc 420
   gctcgccgtc atgacgcact cgccggtatg agctgcaaaa tcgactacgt caagaccgac 480
   ttctgcaaca tgagcttagc cgacaacacc ttcgacggcg cctacgccat cgaggccaca 540
   tgccacgcaa aggacaaggt caagtgctat agcgaggtct tccgtgtcat caagcccggc 600
   acctgcttcg tcctgtacga gtggtgcatg accgacaagt acaaccccaa tgacgagtac 660
   catcgcacga tcaagcaccg cattgagctg ggcgacggcc tgccggagat ggagacgtgc 720
   aagcaggtga tcgagtacat gaaggaggcc ggtttcgtgg tggaggaagc catagatgtc 780
   atcagtcagt tcgagtccag ccccatcaag agcatcccgt ggtaccagcc gctggttggc 840
   gactactcgt ccctgcaggg cctgcgctct accccgattg gccgcatcct caccaacatc 900
   atgtgtcgcg tgctggagtt cgtgcaccta gctccgaagg gcacgtacaa ggcgacggag 960
   gttttggagg aggctgcgga aagcctggtg gtgggcggtc agctcggcat cttcacgccg 1020
   tccttctaca tccgcgctcg caagccgtcc aagcaggcc                        1059
<210> 13
   <211> 666
   <212> PRT
   <213> Artificial Sequence
<220>
   <223> NS fusion polypeptide
<400> 13
   Met Pro Arg Lys Ile Ile Leu Asp Cys Asp Pro Gly Ile Asp Asp Ala
    1               5                  10                  15
   Val Ala Ile Phe Leu Ala His Gly Asn Pro Glu Val Glu Leu Leu Ala
               20                  25                  30
   Ile Thr Thr Val Val Gly Asn Gln Thr Leu Glu Lys Val Thr Arg Asn
           35                  40                  45
   Ala Arg Leu Val Ala Asp Val Ala Gly Ile Val Gly Val Pro Val Ala
       50                  55                  60
   Ala Gly Cys Thr Lys Pro Leu Val Arg Gly Val Arg Asn Ala Ser Gln
   65                  70                  75                  80
   Ile His Gly Glu Thr Gly Met Gly Asn Val Ser Tyr Pro Pro Glu Phe
                   85                  90                  95
   Lys Thr Lys Leu Asp Gly Arg His Ala Val Gln Leu Ile Ile Asp Leu
               100                 105                 110
   Ile Met Ser His Glu Pro Lys Thr Ile Thr Leu Val Pro Thr Gly Gly
           115                 120                 125
   Leu Thr Asn Ile Ala Met Ala Val Arg Leu Glu Pro Arg Ile Val Asp
       130                 135                 140
   Arg Val Lys Glu Val Val Leu Met Gly Gly Gly Tyr His Thr Gly Asn
   145                 150                 155                 160
   Ala Ser Pro Val Ala Glu Phe Asn Val Phe Val Asp Pro Glu Ala Ala
                   165                 170                 175
   His Ile Val Phe Asn Glu Ser Trp Asn Val Thr Met Val Gly Leu Asp
               180                 185                 190
   Leu Thr His Gln Ala Leu Ala Thr Pro Ala Val Gln Lys Arg Val Lys
           195                 200                 205
   Glu Val Gly Thr Lys Pro Ala Ala Phe Met Leu Gln Ile Leu Asp Phe
       210                 215                 220
   Tyr Thr Lys Val Tyr Glu Lys Glu Arg Asn Thr Tyr Ala Thr Val His
   225                 230                 235                 240
   Asp Pro Cys Ala Val Ala Tyr Val Ile Asp Pro Thr Val Met Thr Thr
                   245                 250                 255
   Glu Gln Val Pro Val Asp Ile Glu Leu Asn Gly Ala Leu Thr Thr Gly
               260                 265                 270
   Met Thr Val Ala Asp Phe Arg Tyr Pro Arg Pro Lys His Cys His Thr
           275                 280                 285
   Gln Val Ala Val Lys Leu Asp Phe Asp Lys Phe Trp Cys Leu Val Ile
       290                 295                 300
   Asp Ala Leu Lys Arg Ile Gly Asp Pro Gln Ser Ala Gly Gly Arg Glu
   305                 310                 315                 320
   Thr Ala Pro Thr Asn Leu Ile Arg Arg Arg Asn Lys Asp Glu Thr Asn
                   325                 330                 335
   Gly Asp Val Ser Ala Ala Ala Asp Arg Phe Arg Asp Arg Phe Glu Lys
               340                 345                 350
   Ala Thr Leu Glu Glu Arg Lys Ala Ala Thr Thr Thr Met Val Asn Glu
           355                 360                 365
   Tyr Tyr Asp Leu Val Thr Asp Phe Tyr Glu Tyr Gly Trp Gly Gln Asn
       370                 375                 380
   Phe His Phe Ala Pro Arg Tyr Ala Gly Glu Thr Phe Phe Glu Ser Leu
   385                 390                 395                 400
   Ala Arg His Glu Tyr Phe Leu Ala Ala Arg Gly Gly Phe Met Glu Gly
                   405                 410                 415
   Asp His Ile Val Asp Val Gly Cys Gly Val Gly Gly Pro Ala Arg Asn
               420                 425                 430
   Met Val Arg Leu Thr Arg Cys Asn Val Ile Gly Val Asn Asn Asn Asp
           435                 440                 445
   Tyr Gln Ile Ser Arg Ala Arg Arg His Asp Ala Leu Ala Gly Met Ser
       450                 455                 460
   Ser Lys Ile Asp Tyr Val Lys Thr Asp Phe Cys Asn Met Ser Leu Ala
   465                 470                 475                 480
   Asp Asn Thr Phe Asp Gly Ala Tyr Ala Ile Glu Ala Thr Cys His Ala
                   485                 490                 495
   Lys Asp Lys Val Lys Cys Tyr Ser Glu Val Phe Arg Val Ile Lys Pro
               500                 505                 510
   Gly Thr Cys Phe Val Leu Tyr Glu Trp Cys Met Thr Asp Lys Tyr Asn
           515                 520                 525
   Pro Asn Asp Glu Tyr His Arg Thr Ile Lys His Arg Ile Glu Leu Gly
       530                 535                 540
   Asp Gly Leu Pro Glu Met Glu Thr Cys Lys Gln Val Ile Glu Tyr Met
   545                 550                 555                 560
   Lys Gln Ala Gly Phe Val Val Glu Glu Ala Ile Asp Val Ile Ser Gln
                   565                 570                 575
   Phe Glu Ser Ser Pro Ile Lys Ser Ile Pro Trp Tyr Gln Pro Leu Val
               580                 585                 590
   Gly Asp Tyr Ser Ser Leu Gln Gly Leu Arg Ser Thr Pro Ile Gly Arg
           595                 600                 605
   Ile Leu Thr Asn Val Met Cys Arg Val Leu Glu Phe Val Arg Leu Ala
       610                 615                 620
   Pro Lys Gly Thr Tyr Lys Ala Thr Glu Ile Leu Glu Glu Ala Ala Glu
   625                 630                 635                 640
   Ser Leu Val Val Gly Gly Gln Leu Gly Ile Phe Thr Pro Ser Phe Tyr
                   645                 650                 655
   Ile Arg Ala Arg Lys Pro Ser Lys Gln Ala
               660                 665
<210> 14
   <211> 2001
   <212> DNA
   <213> Artificial Sequence
<220>
   <223> Nucleic acid sequence encoding NS fusion polypeptide
<400> 14
   atgccgcgca agattattct cgattgtgat cccgggatcg atgatgccgt ggccatcttt 60
   ctcgcccacg gcaacccgga ggtcgagctg ctggccatta cgacggtggt gggcaaccag 120
   accctggaga aggtgacccg gaacgcgcgg ctggtagctg acgtagccgg catcgttggt 180
   gtgcccgtcg cggctggttg caccaagccc ctcgtgcgcg gtgtgcggaa tgcctctcag 240
   attcatggcg aaaccggcat gggtaacgtc tcctacccac cagagttcaa gacaaagttg 300
   gacggccgtc atgcagtgca gctgatcatc gaccttatca tgtcgcacga gccgaagacg 360
   atcacgcttg tgcctacggg tggcctgacg aacattgcga tggctgtccg tcttgagccg 420
   cgcatcgtgg accgtgtgaa ggaggtggtt ctgatgggtg gcggctacca tactggtaat 480
   gcgtcccctg tagcggagtt caacgtcttc gtcgacccgg aggcggcgca cattgtgttc 540
   aacgagagct ggaacgtaac gatggtgggg ctggacctaa cgcaccaggc actcgccacg 600
   ccggcggtcc agaagcgagt gaaggaggtg ggcacgaagc cggctgcctt catgctgcag 660
   attttggact tttacacgaa ggtgtacgaa aaggagcgca acacgtacgc gacggtgcac 720
   gatccctgcg ctgtggcgta cgtgattgac cccaccgtga tgacgacgga gcaagtgcca 780
   gtggacatcg agctcaatgg ggcactgacg actgggatga cggtcgcgga cttccgctac 840
   ccacggccaa agcactgcca cacgcaggtg gctgtgaagc tggacttcga caagttttgg 900
   tgcctcgtga ttgacgcact caagcgcatc ggcgatcctc aatccgccgg tggccgtgag 960
   accgcgccga cgaacctgat tcgtcgccgc aacaaggacg agacaaacgg ggatgtcagc 1020
   gccgccgccg accgcttccg cgaccgcttc gagaaggcaa ccctcgagga gcgcaaggcc 1080
   gccaccacga cgatggtcaa cgagtactac gacctggtga cggacttcta cgagtacggc 1140
   tggggccaga acttccattt cgcgccgcgc tacgccggcg agaccttctt cgagtccctc 1200
   gcgcgccacg agtacttcct ggccgctcgc ggcggcttca tggagggcga ccacatcgtc 1260
   gacgtgggct gcggcgtcgg cggtccggcg cgcaacatgg ttcgcctcac gcgctgcaac 1320
   gtcatcggcg tcaacaacaa cgattaccag atcagccgcg ctcgccgtca tgacgcgctc 1380
   gccggtatga gctccaagat cgactacgtc aagaccgact tctgcaacat gagcttagcc 1440
   gacaacacct tcgacggcgc ctacgccatc gaggccacct gccacgcaaa ggacaaggtc 1500
   aagtgctata gcgaggtctt ccgtgtcatc aagcccggca cctgctttgt cctgtacgag 1560
   tggtgcatga ccgacaagta caaccccaat gacgagtacc accgcacaat caagcaccgc 1620
   atcgagctgg gcgacggcct gccggagatg gagacgtgca aacaggtgat cgagtacatg 1680
   aagcaggccg gcttcgtggt ggaggaggcc atagacgtca tcagtcagtt cgagtccagc 1740
   cccatcaaga gtatcccgtg gtaccagccg ctggtcggcg actattcgtc cctgcagggc 1800
   ctgcgctcta ccccgattgg ccgcatcctc acgaacgtca tgtgtcgcgt gctggagttc 1860
   gtgcgcctag ctccgaaggg cacgtacaag gcgacggaga ttttggagga ggctgcggaa 1920
   agcctggtgg tgggcggtca gctcggcatc ttcacgccgt ccttctacat ccgcgctcgc 1980
   aagccgtcca agcaggctta g                                           2001